# EUROPEAN PATENT APPLICATION

(11) **EP 4 480 490 A1**
(43) Date of publication of application: **25.12.2024**
(21) Application number: 23181134.0
(22) Date of filing: 23.06.2023
(51) Int. Cl.: A61K 38/17, A61P 9/00, A61P 9/04, A61P 9/10

(54) **BRICK1 FOR USE IN TREATING MYOCARDIAL DISEASES**

(71) Applicant: Medizinische Hochschule Hannover, 30625 Hannover (DE)
(72) Inventor: WOLLERT, Kai Christoph, 30177 Hannover (DE); KORF-KLINGEBIEL, Moritmer, 37115 Duderstadt (DE); SANDU, Mircea-Andrei, 30625 Hannover (DE); POLTEN, Felix, 30161 Hannover (DE)
(74) Representative: Zwicker, Jörk

(57) **Abstract**

The present invention pertains to a polypeptide comprising the amino acid sequence according to SEQ ID NO: 1 or a variant or fragment thereof for use in treating or preventing heart failure, myocardial infarction or hypertrophy, reducing infarct size or scar size after myocardial infarction, inducing endothelial cell sprouting after myocardial infarction, protecting cells from ischemia- or reperfusion-induced injury, promoting angiogenesis after myocardial infarction, promoting coronary endothelial cell migration and proliferation, inhibiting cardiomyocyte hypertrophy, or inhibiting inflammation. The present invention further provides a nucleic acid sequence encoding the polypeptide, vectors comprising the nucleic acid sequence, host cells comprising the nucleic acid or vector, and respective pharmaceutical compositions for the same use.

## Description

The present invention relates to a protein comprising SEQ ID NO: 1 and its use in medicine, specifically its use in treating and preventing adverse conditions of the heart.

### Background of the invention

Heart failure (HF) is a clinical syndrome developing in response to myocardial stress (e.g. acute myocardial infarction (acute MI)), hemodynamic overload, valvular heart disease, metabolic stress, or genetic mutations (Metra M, Teerlink JR. Heart failure. Lancet. 2017; 390:1981-1995). At the cellular level, these stressors trigger maladaptive cardiac hypertrophy, characterized by cardiomyocyte hypertrophy, cardiomyocyte death, interstitial matrix remodeling, and capillary rarefication leading to systolic or diastolic dysfunction; *i.e.* heart failure with reduced ejection fraction (HFrEF), heart failure with mildly reduced ejection fraction (HFmrEF), or heart failure with preserved ejection fraction (HFpEF).

Although progress has been made in the treatment of HF, patients with advanced HFrEF or those with HFpEF continue to have a poor prognosis, and there is an urgent need to develop new disease-modifying therapies (Metra and Teerlink 2017). Acute MI remains a leading cause of HF. MI is caused by a thrombotic occlusion of a coronary artery, leading to progressive cell death in the non-perfused myocardium. Even if rapid reperfusion of the infarct-related artery by angioplasty and stent implantation can salvage ischemic myocardium, many patients still experience a massive loss of cardiomyocytes. MI triggers an inflammatory response that leads to a replacement of the necrotic area with granulation tissue and eventually a collagen-rich scar. Loss of viable tissue and scar formation induce profound alterations of cardiac tissue architecture leading to cardiac chamber dilatation and HF (Anderson JL, Morrow DA. Acute myocardial infarction. N Engl J Med. 2017; 376:2053-2064).

While rapid reperfusion therapy has improved outcomes in patients with acute MI, especially patients with large MIs continue to have a poor prognosis. Outcome in acute MI may be further improved by reducing cardiomyocyte cell death after reperfusion (by targeting reperfusion injury) and/or by improving wound healing (e.g. by enhancing angiogenesis in the infarct wound). Both of these approaches are expected to reduce scar size and the risk of developing chronic HF (Heusch G. Myocardial ischaemia-reperfusion injury and cardioprotection in perspective. Nat Rev Cardiol. 2020; 17:773-789; Wu X, Reboll MR, Korf-Klingebiel M, Wollert KC. Angiogenesis after acute myocardial infarction. Cardiovasc Res. 2021; 117:1257-1273).

The present inventors identified two different polypeptides exhibiting pro-angiogenic and/or cytoprotective effects secretome analysis in bone marrow cells obtained from patients with acute MI, which proteins were named Factor 1 (MYDGF) (Korf-Klingebiel M et al., Nat Med. 2015; 21:140-149) and Factor 2 (Reboll MR et al., Circulation 2017; 136:1809-1823).

There remains a need in the art for further proteins and compounds for treating and preventing adverse conditions of the heart.

BRICK1 (UniProt entry: Q8WLJW1) is an evolutionary conserved 75 amino acid polypeptide encoded by a small open reading frame on chromosome 3 in humans (gene ID: 55845). In plants and animals, BRICK1 is known as a subunit of the intracellular WAVE regulatory complex that promotes actin branching (Frank MJ, Smith LG. A small, novel protein highly conserved in plants and animals promotes the polarized growth and division of maize leaf epidermal cells. Curr Biol. 2002; 12:849-853; EdenS, Rohatgi R, Podtelejnikov AV, Mann M, Kirschner MW. Mechanism of regulation of WAVE1-induced actin nucleation by Rac1 and Nck. Nature. 2002; 418:790-793; Takenawa T, Suetsugu S. The WASP-WAVE protein network: connecting the membrane to the cytoskeleton. Nat Rev Mol Cell Biol. 2007; 8:37-48). The present inventors surprisingly found that BRICK1 can be released into the extracellular space where it behaves as a growth factor and acts on target cells in an autocrine and paracrine manner. The use of BRICK1 and variants thereof in treating and preventing adverse conditions of the heart is described herein for the first time.

### Summary of the invention

The present invention provides a polypeptide comprising the amino acid sequence according to SEQ ID NO: 1 or a variant thereof, or a fragment of SEQ ID NO: 1 or of the variant thereof, for use in treating or preventing heart failure, myocardial infarction or hypertrophy, wherein the variant has at least 80% sequence identity to SEQ ID NO: 1, wherein the fragment comprises at least amino acids 14 to 75 of SEQ ID NO: 1 or of the variant thereof.

According to one embodiment, the fragment or the variant exhibits the biological function of BRICK1. According to a preferred embodiment, the biological function of BRICK1 is determined by determining the effect of the polypeptide on endothelial cell migration, preferably in the scratch assay described herein below.

According to a further aspect, the present invention provides a polypeptide comprising the amino acid sequence according to SEQ ID NO: 1 or a variant thereof, or a fragment of SEQ ID NO: 1 or of the variant thereof, for use in reducing infarct size or scar size after myocardial infarction, inducing endothelial cell expansion, protecting cells from ischemia- or reperfusion-induced injury, promoting angiogenesis after myocardial infarction, promoting coronary endothelial cell migration and proliferation, inhibiting cardiomyocyte hypertrophy, or inhibiting inflammation, wherein the variant has at least 80% sequence identity to SEQ ID NO: 1, and wherein the fragment comprises at least amino acids 14 to 75 of SEQ ID NO: 1 or of the variant thereof.

According to one embodiment, the fragment or the variant exhibits the biological function of BRICK1. According to a preferred embodiment, the biological function of BRICK1 is determined by determining the effect of the polypeptide on endothelial cell migration, preferably in the scratch assay described herein.

According to another embodiment, the polypeptide consists of SEQ ID NO: 1. According to another embodiment, the variant of SEQ ID NO: 1 has at least 90% sequence identity to SEQ ID NO: 1 and exhibits the biological function of BRICK1. According to another embodiment, the variant has at least 50% of the biological activity of BRICK1. According to another embodiment, a fragment of the polypeptide or variant thereof comprises at least amino acids 14 to 75.

According to a further embodiment, the cells to be protected from ischemia- or reperfusion-induced injury are cardiomyocytes and/or endothelial cells, preferably coronary endothelial cells.

According to one embodiment, the polypeptide is a recombinant polypeptide.

According to a further aspect, the present invention provides a nucleic acid encoding the polypeptide for use of the invention. According to a preferred embodiment, the nucleic acid is for use in treating or preventing heart failure, myocardial infarction or hypertrophy. According to a further preferred embodiment, the nucleic acid is for use in reducing infarct size or scar size after myocardial infarction, inducing endothelial cell expansion after myocardial infarction, protecting cells from ischemia- or reperfusion-induced injury, promoting angiogenesis after myocardial infarction, promoting coronary endothelial cell migration and proliferation, inhibiting cardiomyocyte hypertrophy, or inhibiting inflammation.

According to a further aspect, the present invention provides a vector comprising a nucleic acid encoding the polypeptide for use of the invention. According to a preferred embodiment, the vector is for use in treating or preventing heart failure, myocardial infarction or hypertrophy. According to a further preferred embodiment, the vector is for use in reducing infarct size or scar size after myocardial infarction, inducing endothelial cell expansion after myocardial infarction, protecting cells from ischemia or from reperfusion injury, promoting angiogenesis after myocardial infarction, promoting coronary endothelial cell migration and proliferation, inhibiting cardiomyocyte hypertrophy, or inhibiting inflammation.

According to a preferred embodiment, the vector is an adenoviral vector.

According to a further aspect, the present invention provides a host cell comprising the nucleic acid or the vector of the invention for use in treating or preventing heart failure, myocardial infarction or hypertrophy. According to a further aspect, the present invention provides a host cell comprising the nucleic acid or the vector of the invention for use in reducing infarct size or scar size after myocardial infarction, inducing endothelial cell expansion after myocardial infarction, protecting cells from ischemia- or reperfusion-induced injury, promoting angiogenesis after myocardial infarction, promoting coronary endothelial cell migration and proliferation, inhibiting cardiomyocyte hypertrophy, or inhibiting inflammation.

According to a further aspect, the present invention provides a pharmaceutical composition comprising the polypeptide, the nucleic acid, the vector, or the host cell for use according to the invention, and optionally a suitable pharmaceutical excipient. According to a preferred embodiment, the pharmaceutical composition is for use in treating or preventing heart failure, myocardial infarction or hypertrophy. According to a further preferred embodiment, the pharmaceutical composition is for use in reducing infarct size or scar size after myocardial infarction, inducing endothelial cell expansion after myocardial infarction, protecting cells from ischemia- or reperfusion-induced injury, promoting angiogenesis after myocardial infarction, promoting coronary endothelial cell migration and proliferation, inhibiting cardiomyocyte hypertrophy, or inhibiting inflammation.

According to yet another embodiment, the pharmaceutical composition is administered through the oral, intravenous, subcutaneous, intramucosal, intraarterial, intramuscular or intracoronary route.

According to a further embodiment, the administration is through one or more bolus injection(s) and/or infusion(s).

Further aspects and embodiments of the invention will become apparent from the appending claims and the following detailed description.

### Description of the figures

The invention is further illustrated by the following figures and examples without being limited thereto.

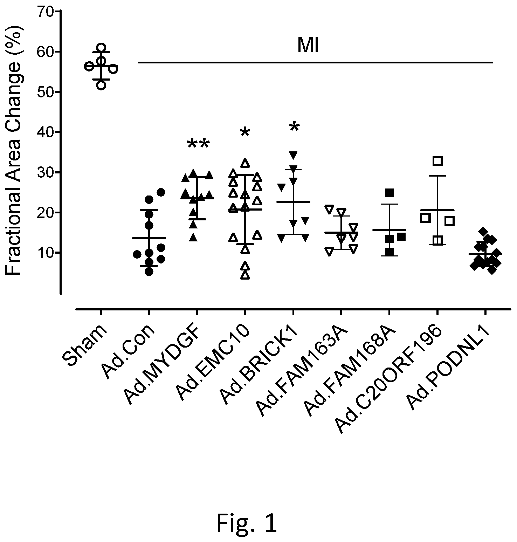

**Fig. 1** shows the results of an adenoviral in vivo screen of five candidate factors (BRICK1, FAM163A, FAM168A, SHLD1/C20orf196, PODNL1) identified in a bioinformatic secretome analysis in bone marrow cells from patients with acute MI. BRICK1 is here identified as a protein that enhances heart function after acute myocardial infarction. *p<0.05, **p<0.01 vs control virus (Ad.Con).

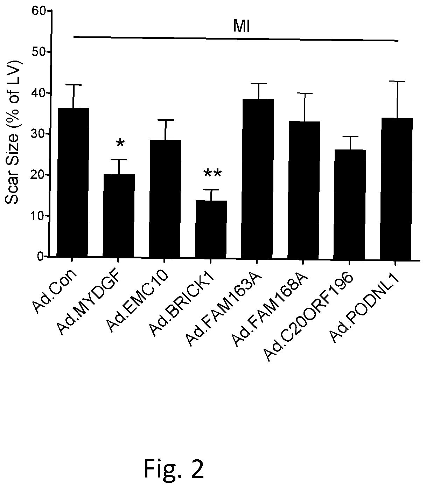

**Fig. 2** shows scar size reduction after acute myocardial infarction. *p<0.05, **p<0.01 vs Ad.Con.

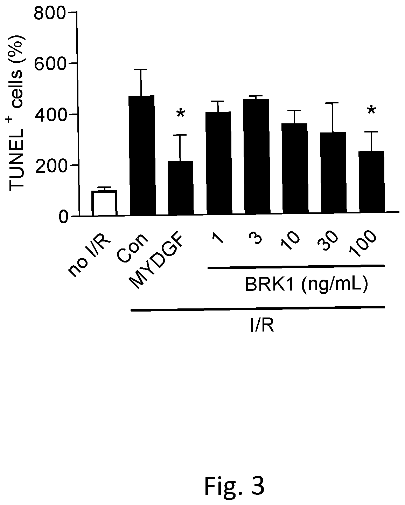

**Fig. 3** shows BRICK1 and MYDGF effects on simulated ischemia/reperfusion (I/R)-induced cell death of neonatal rat ventricular cardiomyocytes. *P < 0.05 vs. I/R control (Con).

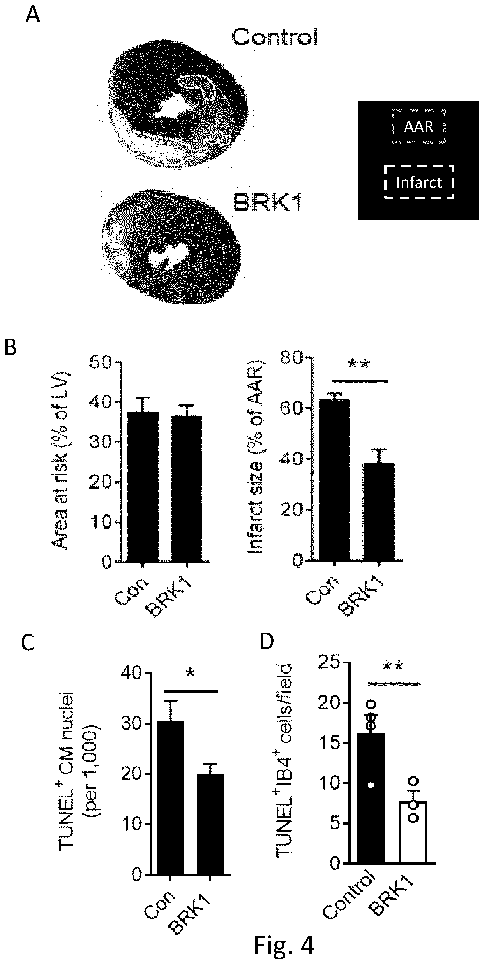

**Fig. 4** shows area at risk and infarct size of basal, midventricular, and apical LV tissue slices after inducing myocardial infarction in mice and administering recombinant mouse BRICK1 (or phosphate-buffered control medium only, Con). (A) tissue slices; (B) to (D) show data summary on area at risk and infarct size (B), number of apoptotic cardiac myocyte nuclei in the infarct border zone 24 hours after reperfusion (C), and number of apoptotic endothelial cell nuclei in the infarct border zone 24 hours after reperfusion (D). *P < 0.05, **P < 0.01.

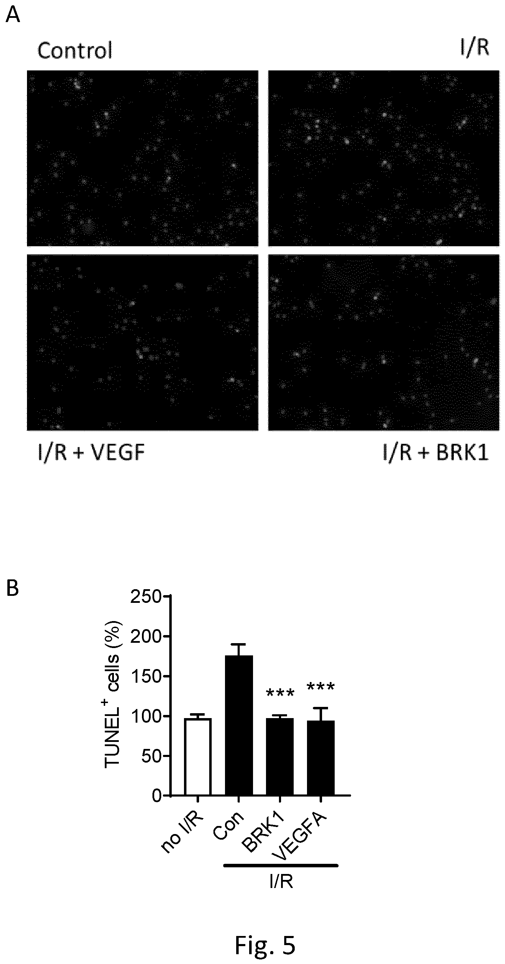

**Fig. 5** shows human coronary artery endothelial cells (HCAECs) cultured in the absence (control) or presence of recombinant human BRICK1 or vascular endothelial growth factor (VEGFA) and exposed to simulated ischemia and reperfusion (I/R). (A) microscopic DAPI (blue) and TUNEL (red) stainings. (B) summary of data. * * *P < 0.001 vs. unstimulated I/R control (Con).

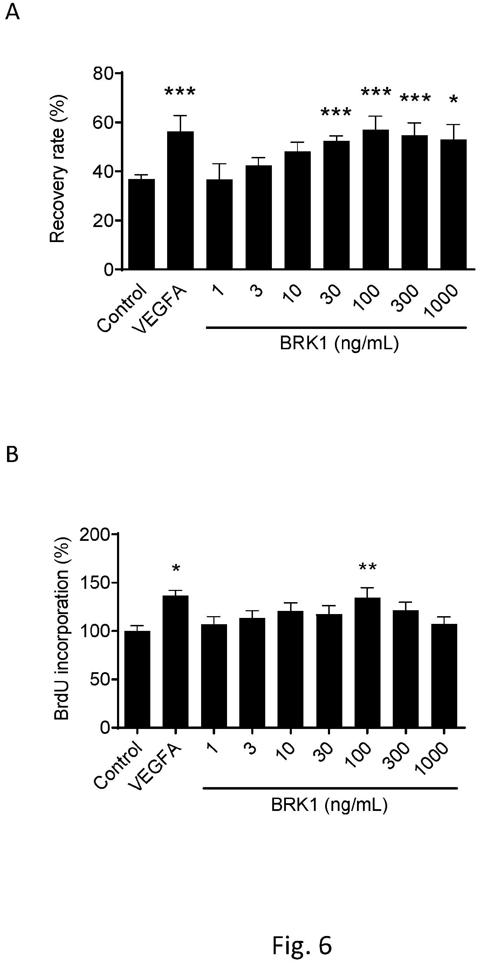

**Fig. 6** shows the effect of human BRICK1 on human coronary artery endothelial cell (HCAEC) migration and proliferation. (A) Confluent HCAEC monolayers were scratched with a pipet tip. Microscopic images were taken at baseline (0 h) and after 16 h. Recovery rate (%) was calculated as ([cell free area at 0 h - cell free area at 16 h] / cell free area at 0 h) × 100, providing a percentage value for recovery rate and thus an index of cell migration; n=4 experiments. (B) BrdU incorporation after stimulation for 48 h as a measure for cell proliferation. *P < 0.05, **P < 0.01, ***P < 0.001 vs unstimulated control.

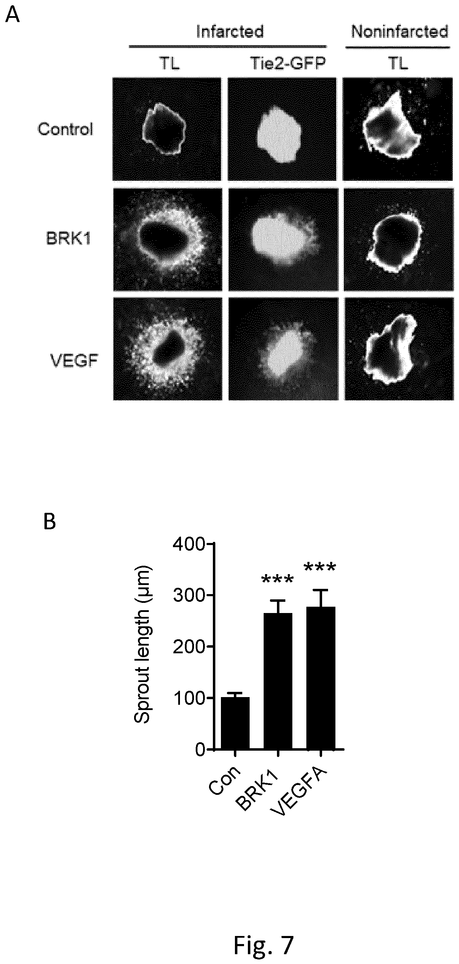

**Fig. 7** shows induction of cell sprouting in endothelial cells obtained from murine myocardial infarct explants. (A) Exemplary images (TL, transmitted light; GFP fluorescence). (B) Graphic summary of the data showing sprout length in µm after stimulation with BRICK1 or VEGFA (n=5 experiments per group). ***P < 0.001 vs. unstimulated control (Con).

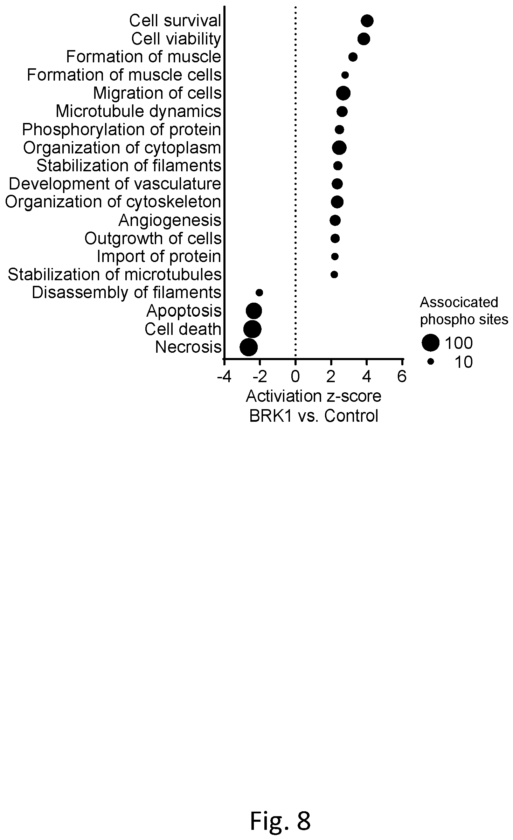

**Fig. 8** shows phosphoproteome changes in human coronary artery endothelial cells (HCAEC) associated with cell viability and reduced cell death induced by BRICK1 stimulation for 15 min. Analysis based on all significantly regulated phosphosites (P < 0.05 vs. control).

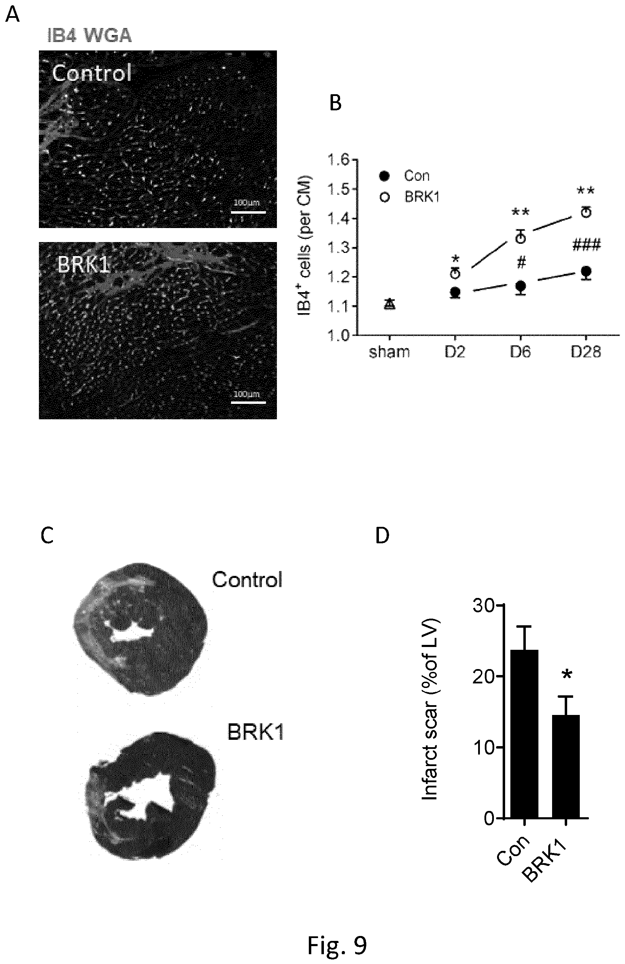

**Fig. 9** shows treatment effects of BRICK1 on angiogenesis and scar size. (A) fluorescent microscopy images of capillary density quantification (isolectin B4⁺ cells per cardiomyocyte, CM) in the infarct border zone 28 days (28D) after myocardial infarction. (B) Summary of data obtained from microscopic images, 2 days (2D), 6 days (2D), and 28 days (28D) after myocardial infarction. *P < 0.05, **P < 0.01 vs. sham-operated, untreated mice. ^{#}P < 0.05, ^{###}P < 0.001 BRICK1 vs. phosphate-buffered saline treatment (control, Con). (C) LV scar size determined by Masson's trichome staining 28 days after reperfusion. (D) Data summary of scar size determination. *P < 0.05.

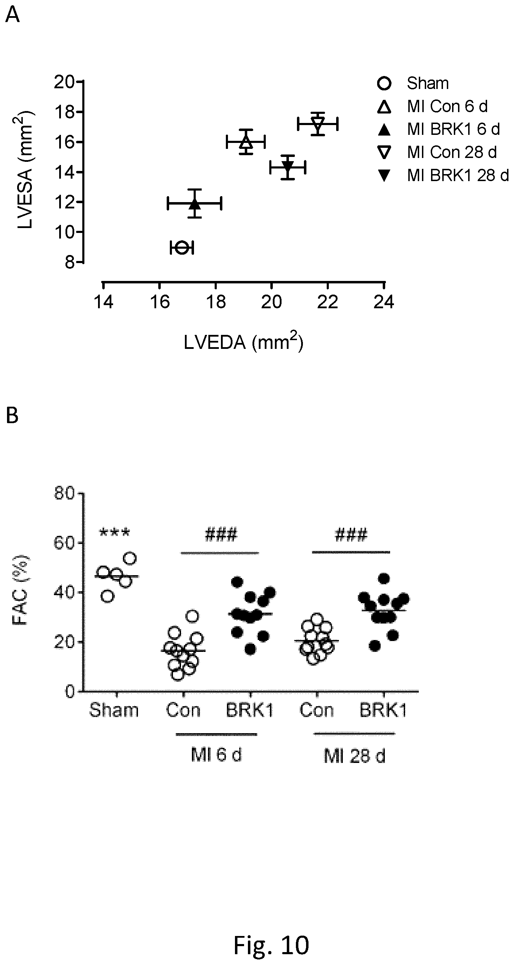

**Fig. 10** shows treatment effects of BRICK1 on left ventricular remodelling and LV function after MI. (A) LV end-diastolic area (LVEDA) and LV end-systolic area (LVESA) recorded by high-resolution echocardiography at 6 days and 28 days after myocardial infarction. Values from sham-operated, untreated mice are shown as well. (B) Calculation of fractional area change (FAC) as [(LVEDA - LVESA) / LVEDA] × 100% as a measure of systolic function. ***P < 0.001 vs. all MI groups; ^{###}P < 0.001 (BRICK1 vs. phosphate-buffered saline treatment [control, Con]).

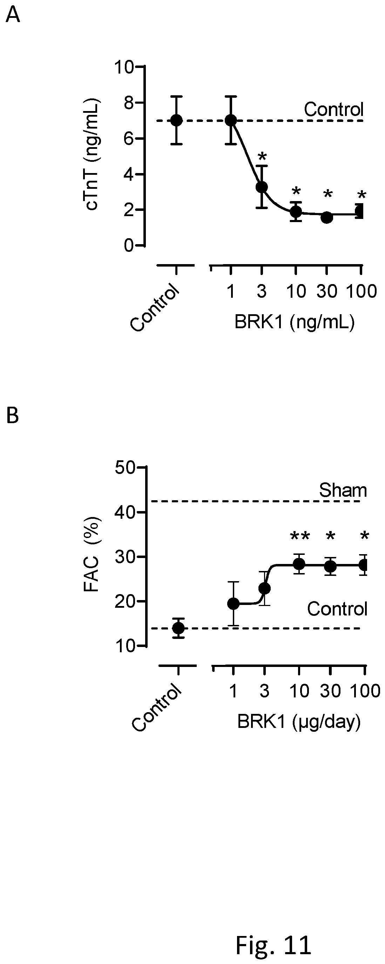

**Fig. 11** shows dose-dependent effects of BRICK1 on (A) cardiac troponin T levels, and on (B) fractional area change in myocardial infarction mice.

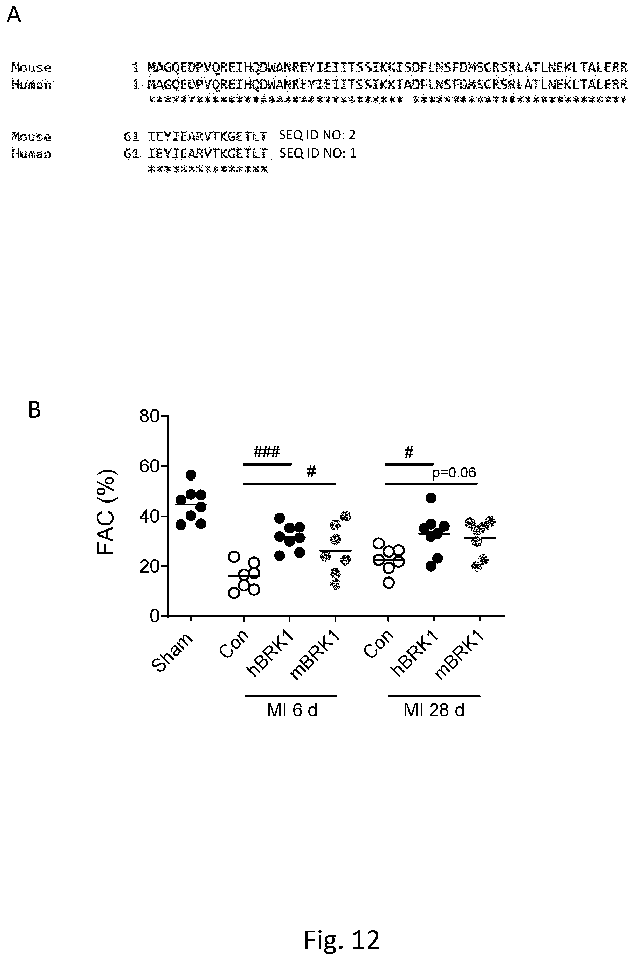

**Fig. 12** (A) shows amino acid sequence comparison of mouse and human BRICK1. (B) shows the effect of mouse (mBRK1) and human BRICK1 (hBRK1) on fractional area change in mice after myocardial infarction.

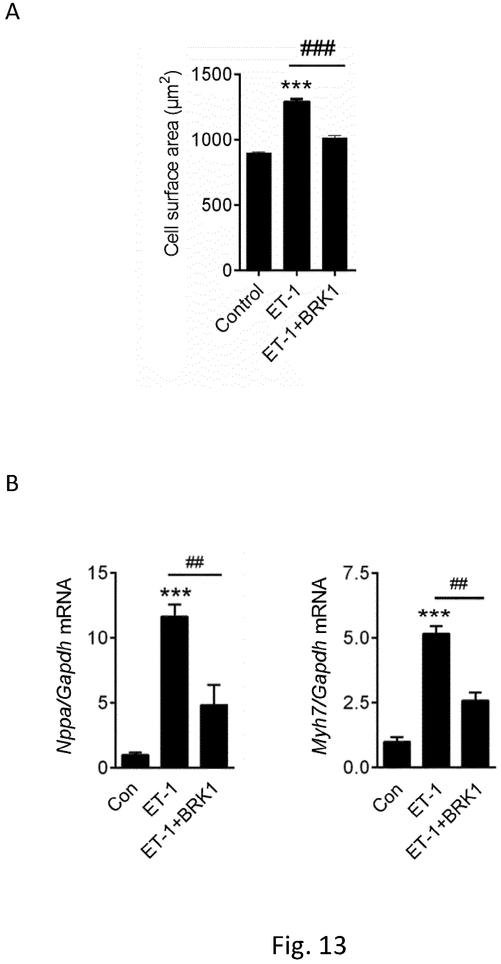

**Fig. 13** shows the effect of BRICK1 on endothelin 1 (ET-1)-induced neonatal rat ventricular cardiomyocyte (NRCM) hypertrophy. (A) Cell size assessed by planimetry after 24 h. (B) Expression levels of maladaptive hypertrophy marker genes *Nppa* mRNA and *Myh7* mRNA and determined after 24 h by RT-qPCR. ***P < 0.001 vs. untreated control; ^{##}P < 0.01, ^{###}P < 0.001.

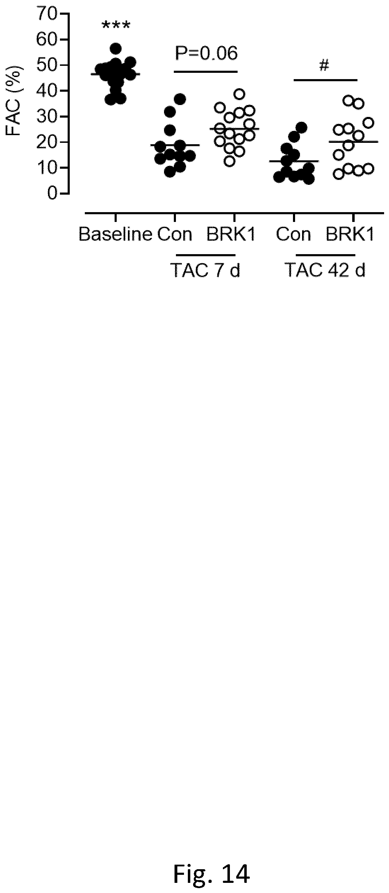

**Fig. 14** shows treatment effect of BRICK1 on left ventricular dysfunction after induced hypertrophy and heart failure in mice after transverse aortic constriction (TAC). Calculation of fractional area change as [(LVEDA - LVESA) / LVEDA] × 100%. ***P < 0.001 vs. all TAC groups; ^{#}P < 0.05.

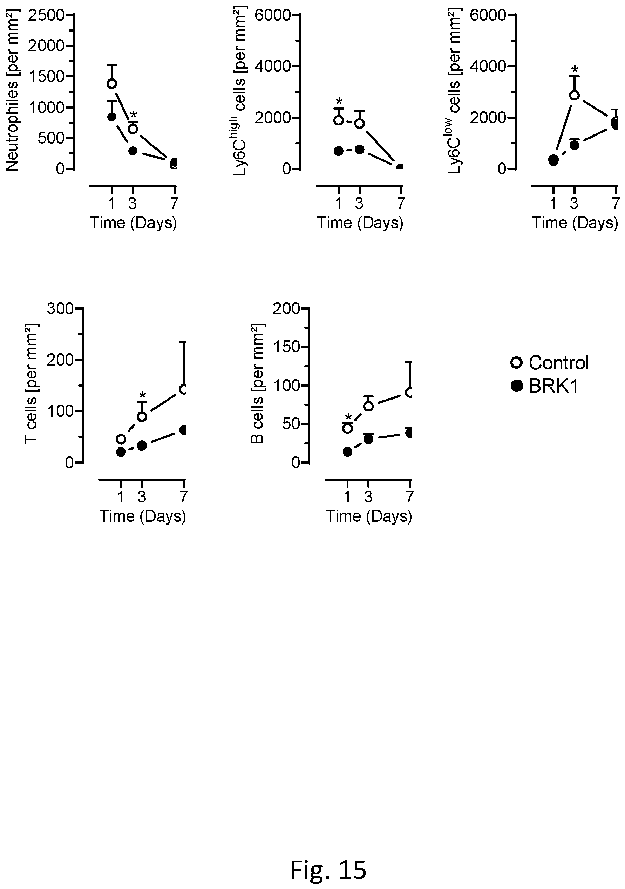

**Fig. 15** shows effects of BRICK1 on inflammatory cell accumulation in the infarct region at 1, 3, and 7 days after myocardial infarction. *P<0.05 vs. same-day control group.

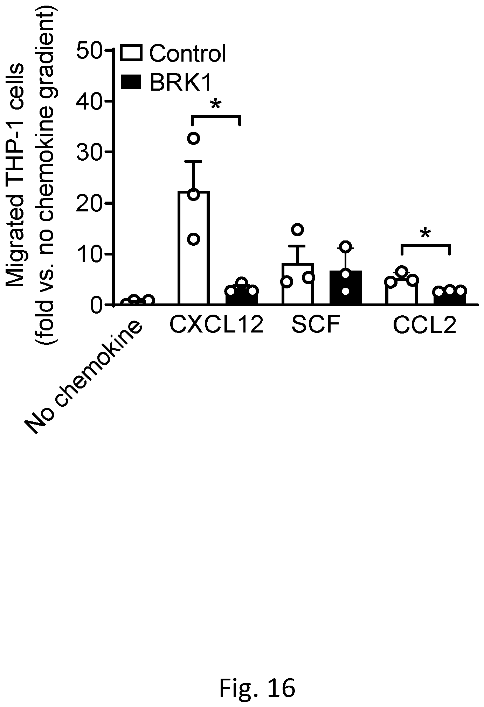

**Fig. 16** shows that BRICK1 inhibits inflammatory cell migration. THP-1 cell migration was induced by chemotactic gradients created by C-X-C motif chemokine 12 (CXCL12), stem cell factor (SCF), or C-C motif chemokine 2 (CCL2). THP-1 cells are a human a monocytic cell line. Incubating the cells with BRICK1 inhibited their transendothelial migratory potential. *P<0.05.

### Sequences

The sequences referred to in the present description are disclosed in the accompanying sequence listing.

SEQ ID NO: 1 is the full-length amino acid sequence of human BRICK1:

MAGQEDPVQREIHQDWANREYIEIITSSIKKIADFLNSFDMSCRSRLATLNEKLTALERRIEYIE
ARVTKGETLT

SEQ ID NO: 2 is the full-length amino acid sequence of mouse BRICK1:

MAGQEDPVQREIHQDWANREYIEIITSSIKKISDFLNSFDMSCRSRLATLNEKLTALERRIEYIE
ARVTKGETLT

SEQ ID NO: 3 is the human BRICK1 cDNA sequence (NCBI Reference Sequence: NM_018462.5):

atggcgggacaggaggatccggtgcagcgggagattcaccaggactgggctaaccgggagtacattgagataatcaccagcagcat
caagaaaatcgcagactttctcaactcgttcgatatgtcttgtcgttcaagacttgcaacactaaacgagaaattgacagcccttgaacggagaataga
gtacattgaagctcgggtgacaaaaggtgagacactcacctag

SEQ ID NO: 4 is the mouse BRICK1 cDNA sequence:

atggcgggacaagaggatccggtgcagcgggagattcaccaggactgggcgaaccgggagtacattgagatcatcaccagcagcat
caagaaaatctcagactttctcaactctttcgatatgtcttgtcgttcaagactcgcaacactaaatgagaaattgacagcccttgaacggagaatagag
tacattgaagcgagggtgacaaagggtgagactctgacctag

### Detailed description of the invention

Before the present invention is described in detail below, it is to be understood that this invention is not limited to the particular methodology, protocols and reagents described herein as these may vary. It is also to be understood that the terminology used herein is for the purpose of describing particular embodiments only, and it is not intended to limit the scope of the present invention which will be limited only by the appended claims. Unless defined otherwise, all technical and scientific terms used herein have the same meanings as commonly understood by one of ordinary skill in the art.

Preferably, the terms used herein are defined as described in "A multilingual glossary of biotechnological terms: (IUPAC Recommendations)", Leuenberger, H.G.W, Nagel, B. and Klbl, H. eds. (1995), Helvetica Chimica Acta, CH-4010 Basel, Switzerland).

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integers or steps. In the following passages, different aspects of the invention are defined in more detail. Each aspect so defined may be combined with any other aspect or aspects unless clearly indicated to the contrary. Any feature indicated as being optional, preferred or advantageous may be combined with any other feature or features indicated as being optional, preferred or advantageous.

Several documents are cited throughout the text of this specification. Each of the documents cited herein (including all patents, patent applications, scientific publications, manufacturer's specifications, instructions etc.), whether supra or infra, is hereby incorporated by reference in its entirety. Nothing herein is to be construed as an admission that the invention is not entitled to antedate such disclosure by virtue of prior invention. Some of the documents cited herein are characterized as being "incorporated by reference". In the event of a conflict between the definitions or teachings of such incorporated references and definitions or teachings recited in the present specification, the text of the present specification takes precedence.

In the following, the elements of the present invention will be described. These elements are listed with specific embodiments; however, it should be understood that they may be combined in any manner and in any number to create additional embodiments. The variously described examples and preferred embodiments should not be construed to limit the present invention to only the explicitly described embodiments. The present description should be understood to support and encompass embodiments which combine the explicitly described embodiments with any number of the disclosed and/or preferred elements. Furthermore, any permutations and combinations of all described elements in this application should be considered disclosed by the description of the present application unless the context indicates otherwise.

### Definitions

In the following, some definitions of terms frequently used in this specification are provided. These terms will, in each instance of its use, in the remainder of the specification have the respectively defined meaning and preferred meanings.

As used in this specification and the appended claims, the singular forms "a", "an", and "the" include plural referents, unless the content clearly dictates otherwise.

The term "sequence comparison" is used herein to refer to the process wherein one sequence acts as a reference sequence, to which test sequences are compared. When using a sequence comparison algorithm, test and reference sequences are entered into a computer, if necessary, subsequence coordinates are designated, and sequence algorithm program parameters are designated. Default program parameters are commonly used, or alternative parameters can be designated. The sequence comparison algorithm then calculates the percent sequence identities for the test sequences relative to the reference sequence, based on the program parameters. In case where two sequences are compared and the reference sequence is not specified in comparison to which the sequence identity percentage is to be calculated, the sequence identity is to be calculated with reference to the longer of the two sequences to be compared, if not specifically indicated otherwise. If the reference sequence is indicated, the sequence identity is determined on the basis of the full length of the reference sequence indicated by one of the SEQ ID NOs of the present invention, if not specifically indicated otherwise.

Methods of alignment of sequences for comparison are well known in the art. Optimal alignment of sequences for comparison can be conducted, for example, by the local homology algorithm of Smith and Waterman (Adv. Appl. Math. 2:482, 1970), by the homology alignment algorithm of Needleman and Wunsch (J. Mol. Biol. 48:443, 1970), by the search for similarity method of Pearson and Lipman (Proc. Natl. Acad. Sci. USA 85:2444, 1988), by computerized implementations of these algorithms (e.g., GAP, BESTFIT, FASTA, and TFASTA in the Wisconsin Genetics Software Package, Genetics Computer Group, 575 Science Dr., Madison, Wis.), or by manual alignment and visual inspection (see, e.g., Ausubel et al., Current Protocols in Molecular Biology (1995 supplement)). Algorithms suitable for determining percent sequence identity and sequence similarity are the BLAST and BLAST 2.0 algorithms, which are described in Altschul et al. (Nuc. Acids Res. 25:3389-402, 1977), and Altschul et al. (J. Mol. Biol. 215:403-10, 1990), respectively. Software for performing BLAST analyses is publicly available through the National Center for Biotechnology Information (http://www.ncbi.nlm.nih.gov/). This algorithm involves first identifying high scoring sequence pairs (HSPs) by identifying short words of length W in the query sequence, which either match or satisfy some positive-valued threshold score T when aligned with a word of the same length in a database sequence. T is referred to as the neighborhood word score threshold (Altschul et al., supra). These initial neighborhood word hits act as seeds for initiating searches to find longer HSPs containing them. The word hits are extended in both directions along each sequence for as far as the cumulative alignment score can be increased. Cumulative scores are calculated using, for nucleotide sequences, the parameters M (reward score for a pair of matching residues; always >0) and N (penalty score for mismatching residues; always <0). For amino acid sequences, a scoring matrix is used to calculate the cumulative score. Extension of the word hits in each direction are halted when: the cumulative alignment score falls off by the quantity X from its maximum achieved value; the cumulative score goes to zero or below, due to the accumulation of one or more negative-scoring residue alignments; or the end of either sequence is reached. The BLAST algorithm parameters W, T, and X determine the sensitivity and speed of the alignment. The BLASTN program (for nucleotide sequences) uses as defaults a wordlength (W) of 11, an expectation (E) of 10, M=5, N=-4 and a comparison of both strands. For amino acid sequences, the BLASTP program uses as defaults a wordlength of 3, and expectation (E) of 10, and the BLOSUM62 scoring matrix (see Henikoff and Henikoff, Proc. Natl. Acad. Sci. USA 89:10915, 1989) alignments (B) of 50, expectation (E) of 10, M=5, N=-4, and a comparison of both strands. The BLAST algorithm also performs a statistical analysis of the similarity between two sequences (see, e.g., Karlin and Altschul, Proc. Natl. Acad. Sci. USA 90:5873-87, 1993). One measure of similarity provided by the BLAST algorithm is the smallest sum probability (P(N)), which provides an indication of the probability by which a match between two nucleotide or amino acid sequences would occur by chance. For example, a nucleic acid is considered similar to a reference sequence if the smallest sum probability in a comparison of the test nucleic acid to the reference nucleic acid is less than about 0.2, typically less than about 0.01, and more typically less than about 0.001.

In cases where two sequences are compared and the reference sequence is not specified in comparison to which the sequence identity percentage is to be calculated, the sequence identity is to be calculated with reference to the longer of the two sequences to be compared, if not specifically indicated otherwise. In accordance with the present invention, the percentage identity of variants of SEQ ID NO: 1 is to be determined in relation to the 75 aa sequence of SEQ ID NO: 1.

The term "nucleic acid" and "nucleic acid molecule" are used synonymously herein and are understood as well-accepted in the art, *i.e.* as single or double-stranded oligo- or polymers of deoxyribonucleotide or ribonucleotide bases or both. The term "nucleic acids" as used herein includes not only deoxyribonucleic acids (DNA) and ribonucleic acids (RNA), but also all other linear polymers in which the bases adenine (A), cytosine (C), guanine (G) and thymine (T) or uracil (U) are arranged in a corresponding sequence (nucleic acid sequence). The invention also comprises the corresponding RNA sequences (in which thymine is replaced by uracil), complementary sequences and sequences with modified nucleic acid backbone or 3 'or 5'-terminus. Nucleic acids in the form of DNA are however preferred.

The term "cell" or "host cell" as used herein relates to an intact cell, *i.e.* a cell with an intact membrane that has not released its normal intracellular components such as enzymes, organelles, or genetic material. An intact cell preferably is a viable cell, *i.e.* a living cell capable of carrying out its normal metabolic functions. Preferably said term relates to any cell which can be transfected or transformed with an exogenous nucleic acid.

The term "regulatory nucleic acid sequence" as used herein refers to gene regulatory regions of DNA. In addition to promoter regions, this term encompasses operator regions more distant from the gene as well as nucleic acid sequences that influence the expression of a gene, such as cis-elements, enhancers or silencers. The term "promoter region" as used herein refers to a nucleotide sequence on the DNA allowing a regulated expression of a gene. The promoter region allows regulated expression of the nucleic acid encoding for the respective protein. The promoter region is located at the 5'-end of the gene and thus before the coding region. Both, bacterial and eukaryotic promoters are applicable for the present invention.

The terms "protein" and "polypeptide" are used interchangeably herein and refer to any peptide-bond-linked chain of amino acids, regardless of length or post-translational modification. Proteins usable in the present invention (including protein derivatives, protein variants, protein fragments, protein segments, protein epitopes and protein domains) can be further modified by chemical modification. A respectively chemically modified polypeptide preferably comprises other chemical groups than the 20 naturally occurring amino acids. Examples of such other chemical groups include without limitation glycosylated amino acids and phosphorylated amino acids. Chemical modifications of a polypeptide may provide advantageous properties as compared to the parent polypeptide, e.g. one or more of enhanced stability, increased biological half-life, or increased water solubility. Chemical modifications applicable to the variants usable in the present invention include without limitation: PEGylation, glycosylation of non-glycosylated parent polypeptides, covalent coupling to therapeutic small molecules, like glucagon-like peptide 1 agonists, including exenatide, albiglutide, taspoglutide, DPP4 inhibitors, incretin and liraglutide, or the modification of the glycosylation pattern present in the parent polypeptide. Such chemical modifications applicable to the variants usable in the present invention may occur co- or post-translational.

The term "variant" as used herein refers to a polypeptide which differs in comparison to the polypeptide or fragment thereof from which it is derived by one or more changes in the amino acid sequence. The polypeptide from which a protein variant is derived is also known as the parent polypeptide. Likewise, the fragment from which a protein fragment variant is derived from is known as the parent fragment. Typically, a variant is constructed artificially, preferably by gene-technological means. Typically, the parent polypeptide is a wild-type protein or wild-type protein domain. Further, the variants usable in the present invention may also be derived from homologs, orthologs, or paralogs of the parent polypeptide or from artificially constructed variant, provided that the variant exhibits at least one biological activity of the parent polypeptide. The changes in the amino acid sequence may be amino acid exchanges, insertions, deletions, N-terminal truncations, or C-terminal truncations, or any combination of these changes, which may occur at one or several sites. In preferred embodiments, a variant usable in the present invention exhibits a total number of up to 30 (up to 1, 2, 3, 4, 5, 6, 7, 8, 9, 10, 15, 20, 25, 30) changes in the amino acid sequence (i.e. exchanges, insertions, deletions, N-terminal truncations, and/or C-terminal truncations). The amino acid exchanges may be conservative, and/or semi-conservative, and/or non-conservative. In preferred embodiments, a variant usable in the present invention differs from the protein or domain from which it is derived by up to 1, 2, 3, 4, 5, 6, 7, 8, 9, 10, 11, 12, 13, 14, 15, 16, 17, 18, 19, 20, 25, or 30 amino acid exchanges, preferably conservative amino acid changes.

The term "at least 80% sequence identity" is used throughout the specification with regard to polypeptide and polynucleotide sequence comparisons. This expression preferably refers to a sequence identity of at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 98.7%, or at least 99% to the respective reference polypeptide or to the respective reference polynucleotide.

Fragments of proteins comprise deletions of amino acids, which may be N-terminal truncations, C-terminal truncations or internal deletions or any combination of these. Such variants comprising N-terminal truncations, C-terminal truncations and/or internal deletions are referred to as "fragments" in the context of the present application. A fragment may be naturally occurring (e.g. splice variants) or it may be constructed artificially, preferably by gene-technological means. Preferably, a fragment (or deletion variant) has a deletion of up to 1, 2, 3, 4, 5, 6, 7, 8, 9, 10, 11, 12, 13, 14, 15, 16, 17, 18, 19 or 20 amino acids at the N-terminus and/or the C-terminus and/or internally as compared to the parent or reference polypeptide. A particularly preferred fragment according to the present invention has an N-terminal deletion of not more than 1, 2, 3, 4, 5, 6, 7, 8, 9, 10, 11, 12, or 13 amino acids and thus comprises at least amino acids 14 to 75 of SEQ ID NO: 1 or of the variant thereof.

As used herein, "treat", "treating" or "treatment" of a disease or disorder means accomplishing one or more of the following: (a) reducing the severity of the disorder; (b) limiting or preventing development of symptoms characteristic of the disorder(s) being treated; (c) inhibiting worsening of symptoms characteristic of the disorder(s) being treated; (d) limiting or preventing recurrence of the disorder(s) in patients that have previously had the disorder(s); (e) limiting or preventing recurrence of symptoms in patients that were previously symptomatic for the disorder(s); (f) reduction of mortality after occurrence of a disease or a disorder; (g) healing; and (h) prophylaxis of a disease. As used herein, "prevent", "preventing", "prevention" or "prophylaxis" of a disease or disorder preferably means preventing that such disease or disorder occurs in patient. According to a preferred embodiment, a treatment results in healing of the respective subject. Thus, the term "treatment" as used herein also includes "healing".

The term "inhibiting apoptosis" refers to the ability of proteins, nucleic acids, vectors or pharmaceutical compositions of the invention to prevent a cell or group of cells to enter apoptosis under conditions, in which control cells or groups of cells enter apoptosis. It is well known to the skilled person how to measure whether a cell undergoes apoptosis, e.g. by TUNEL assay.

The terms "subject", "individual" and "patient" are used herein interchangeable and refer to an individual, such as a human, a non-human primate (e.g. chimpanzees and other apes and monkey species); farm animals, such as birds, fish, cattle, sheep, pigs, goats and horses; domestic mammals, such as dogs and cats; laboratory animals including rodents, such as mice, rats and guinea pigs. The term does not denote a particular age or sex. In a particular meaning, the subject is a mammal. According to a particularly preferred embodiment, the subject is a human. The subject can be a healthy subject or a subject suffering from or suspected of having one or more diseases. A subject suffering from or suspected of having one or more diseases is also referred to as a patient. In accordance with the present invention, the subject, preferably a human, preferably suffers from heart failure, myocardial infarction or hypertrophy as detailed herein below.

The description of the embodiments comprises further definitions and explanations of terms used throughout the application. These descriptions and definitions are valid for the whole application unless it is otherwise stated.

### Description of embodiments

The present invention provides a polypeptide comprising the amino acid sequence according to SEQ ID NO: 1 or a variant thereof, or a fragment of SEQ ID NO: 1 or of the variant thereof, for use in medicine.

According to a preferred aspect of the invention, a polypeptide comprising the amino acid sequence according to SEQ ID NO: 1 or a variant thereof, or a fragment of SEQ ID NO: 1 or of the variant thereof, is provided for the use in treating or preventing heart failure, myocardial infarction or hypertrophy.

According to a further aspect, the polypeptide, fragment or variant thereof in accordance with the present invention is provided for use in reducing infarct size or scar size after myocardial infarction, inducing endothelial cell expansion, protecting cells from ischemia- or reperfusion-induced injury, promoting angiogenesis, promoting coronary endothelial cell migration and proliferation, inhibiting cardiomyocyte hypertrophy, or inhibiting inflammation. Preferably, the polypeptide, fragment or variant thereof is provided for use in treating the conditions cited herein of a patient after myocardial infarction, heart failure or hypertrophy has been diagnosed for said patient.

The variant of the polypeptide has at least 80% sequence identity to SEQ ID NO: 1. According to a preferred embodiment, the variant has at least 90%, 91%, 92%, 93%, 94%, 95%, 96%, 97%, 98%, 98.7% or 99% sequence identity to SEQ ID NO: 1. A person skilled in the art is able to decide without undue burden, which positions in the parental polypeptide can be mutated to which extent and which positions have to be maintained to preserve the functionality of the polypeptide. Such information can, for example, be gained from homologues sequences which can be identified, aligned and analyzed by bioinformatic methods well known in the art. Mutations are preferably introduced in those regions of the protein which are not fully conserved between species, preferably mammals. One example of a non-human BRICK1 variant is mouse BRICK1 shown in SEQ ID NO: 2. The present inventors have also shown the suitability of the polypeptide of SEQ ID NO: 2 to treat or inhibit adverse conditions of the heart as described herein. Thus, according to a preferred embodiment, the variant of SEQ ID NO: 1 is SEQ ID NO: 2. In a particularly preferred embodiment of the invention, the polypeptide of the invention comprises, essentially consists or consists of the amino acid sequence SEQ ID NO: 1 or a fragment thereof. According to a further preferred embodiment of the invention, the polypeptide of the invention comprises, essentially consists or consists of the amino acid sequence SEQ ID NO: 2 or a fragment thereof.

The fragment of SEQ ID NO: 1 or of the variant thereof comprises at least amino acids 14 to 75 of SEQ ID NO: 1 or of the variant thereof. Accordingly, a fragment in accordance with a preferred embodiment comprises an N-terminal deletion of 1, 2, 3, 4, 5, 6, 7, 8, 9, 10, 11, 12, or 13 amino acids of the polypeptide as denoted in SEQ ID NO: 1 or of the variant thereof. A particularly preferred fragment comprises amino acids 14 to 75 of SEQ ID NO: 1 or of the variant thereof.

According to a preferred embodiment, the fragment or variant according to the present invention exhibits the biological function of BRICK1. Whether or not a protein, variant or fragment exhibits the biological function of BRICK1 can be determined by any one of the tests described in the examples below. According to the present invention, a peptide or protein exhibits the biological function of BRICK1 if the results obtained with such peptide or protein compared to the results obtained with the BRICK1 of the present invention shown in at least one of the examples presented herein below achieve at least 50%, at least 60%, at least 70%, at least 80%, at least 90%, at least 95% or 100% of the effect reported for BRICK1 over the indicated controls. According to a preferred embodiment, the biological function of BRICK1 is determined using the cell migration assay as described herein. For the cell migration assay, confluent cell monolayers (preferably human coronary artery endothelial cells) are scratched with a pipet tip to the bottom of the Petri dish. Closing of the resulting cell free strip is the recovery rate (%) calculated as ([cell free area at 0 h - cell free area at 16 h] / cell free area at 0 h) × 100, by evaluating microscopic images taken at baseline (0 h) and 16 h after scratching. The resulting percentage value is used for comparing the biological activity of one compound on cell migration to that of another compound. A detailed example of the cell migration assay is provided in Example 6 and results are shown in Fig. 5. In accordance with the present invention, a compound exhibits the biological activity of BRICK1 if it achieves at least 50%, at least 60%, at least 70%, at least 80%, at least 90%, at least 95% or 100% of the effect of the BRICK1 polypeptide according to SEQ ID NO: 1 in the same cell migration assay set-up described herein.

The polypeptide of the present invention may further comprise additional amino acid sequences, e.g. for stabilizing or purifying the resulting polypeptide or protein. Non-limiting examples of such amino acids are His₆-tags, myc-tags, or FLAG-tags.

In some embodiments it is preferred to mutate protease cleavage sites within the polypeptide of the present invention to stabilize the polypeptide (see e.g. Segers et al. Circulation, 2011, Vol. 123: 1306-1315). The skilled person knows how to determine potential proteolytic cleavage sites within a protein. For example, protein sequences can be submitted to websites providing such analysis as, e.g. http://web.expasy.org/peptide_cutter/. If the protein sequence according to SEQ ID NO: 1 is submitted to http://web.expasy.org/peptide_cutter/, the following cleavage sites with lower frequency (less than 10) are determined:

| **Enzyme** | **Amino acid position(s)** |
|---|---|
| Arg-C proteinase | 10, 19, 44, 46, 59, 60, 67 |
| Asp-N endopeptidase | 5, 14, 33, 39 |
| BNPS-Skatole | 16 |
| CNBr | 1, 41 |
| Chymotrypsin-high specificity | 16, 21, 35, 39, 63 |
| Clostripain | 10, 19, 44, 46, 59, 60, 67 |
| Formic acid | 6, 15, 34, 40 |
| Glutamyl endopeptidase | 5, 11, 20, 23, 52, 58, 62, 65, 72 |
| Iodosobenzoic acid | 16 |
| LysC | 30, 31, 53, 70 |
| LysN | 29, 30, 52, 69 |
| NTCB (2-nitro-5-thiocyanobenzoic acid) | 42 |
| Staphylococcal peptidase I | 5, 11, 20, 23, 52, 58, 62, 65, 72 |

The sites identified in the table above may be altered to remove the recognition/cleavage sequence of the respectively identified protease to increase the serum half-life of the protein.

The present inventors show that the polypeptide of the present invention inter alia supports regeneration of damaged heart tissue, prevents or reduces necrosis, reduces (cell) death, prevents apoptosis of cardiomyocytes or endothelial cells, preferably of coronary endothelial cells, supports angiogenesis, reduces left ventricular scar size, and inhibits inflammation after myocardial infarction.

The polypeptide of the present invention can thus be used for treating insufficiency of the heart, specifically acute or chronic insufficiency of the heart, and right or left or global insufficiency of the heart. The polypeptide of the present invention has been further shown to be useful in treating or preventing myocardial infarction, preferably acute myocardial infarction, angina pectoris, and mechanical overload of the heart.

According to a preferred embodiment, the polypeptide of the present invention protects cells and preferably cardiomyocytes and endothelial cells, more preferably endothelial cells of the myocardium, and most preferably coronary endothelial cells. Specifically preferred cells to be protected in accordance with the present invention are cardiomyocytes. According to a preferred embodiment, the cells are protected from ischemia-induced injury or reperfusion-induced injury, preferably after myocardial infarction.

The polypeptide of the present invention can further be used for treating heart failure. The heart failure in accordance with the present invention is preferably chronic heart failure. Heart failure as referred to herein is preferably heart failure with preserved ejection fraction (HFpEF), heart failure with reduced ejection fraction (HFrEF), or heart failure with mildly reduced ejection fraction (HFmrEF). The heart failure or the chronic heart failure is preferably HFpEF or HFrEF, more preferably the HFpEF is Stage C or Stage D HFpEF, or the HFrEF is Stage C or Stage D HFrEF.

The polypeptide of the present invention can further be used for treating hypertrophy of the heart. The hypertrophy in accordance with a preferred embodiment of the present invention is hypertrophy of cardiomyocytes.

According to a preferred embodiment, the polypeptide of the present invention reduces infarct size or scar size after myocardial infarction.

The polypeptide of the present invention can further be used for inducing endothelial cell expansion after myocardial infarction, promoting angiogenesis after myocardial infarction, promoting coronary endothelial cell migration and proliferation, and for treating or inhibiting inflammation.

In another preferred embodiment, the polypeptide according to the present invention is used for inhibiting apoptosis of cells, preferably of cardiomyocytes or endothelial cells, preferably of coronary endothelial cells. Thus, the polypeptide of the present invention exhibits anti-apoptotic potential and protects the cells or tissues from apoptotic cell death. "Protecting" or "cytoprotective effect" in this context means that the extent of apoptotic cell death is reduced in the cells treated with the polypeptide according to the present invention compared to a control by at least 20%, preferably by at least 30%, more preferably by at least 40%, and even more preferably by at least 50%, and most preferably by at least 60%. The skilled person is able to assess cell death, for example by in situ TdT-mediated dUTP nick end-labeling (TUNEL). Other indicators for apoptosis are, for example, a fragmented genome which can be examined, e.g., by DNA laddering (Liu et al., 2005, Circulation 111: 90-96), cytochrome-c release, or caspase 3 activity (Most et al., 2003, J. Biol. Chem. 278:48404-48412). The anti-apoptotic effect of a peptide may be assessed *in vivo* in an experimental heart failure animal model as described in the examples section.

According to a further aspect, the present invention provides a nucleic acid or a plurality of nucleic acids encoding the polypeptide of the present invention. The nucleic acid or plurality of nucleic acids is specifically for use in treating or preventing the conditions discussed herein.

In a preferred embodiment of the invention, the nucleic acid or plurality of nucleic acids further comprises a transcriptional control element or expression control sequences positioned to control expression of the protein. Such a nucleic acid together with control elements is often termed as an expression system. The term "expression system" as used herein refers to a system designed to produce one or more gene products of interest. Typically, such system is designed "artificially", *i.e.* by gene-technological means usable to produce the gene product of interest *in vivo, in vitro or ex vivo.* The term "expression system" further encompasses the expression of the gene product of interest comprising the transcription of the polynucleotides, mRNA splicing, translation into a polypeptide, co- and post-translational modification of a polypeptide or protein as well as the targeting of the protein to one or more compartments inside of the cell, the secretion from the cell and the uptake of the protein in the same or another cell. This general description refers to expression systems for the use in eukaryotic cells, tissues or organisms. Expression systems for prokaryotic systems may differ, wherein it is well known in the art, how an expression system for prokaryotic cells is constructed.

Regulatory elements present in a gene expression cassette generally include: (a) a promoter transcriptionally coupled to a nucleotide sequence encoding the polypeptide, (b) a 5' ribosome binding site functionally coupled to the nucleotide sequence, (c) a terminator joined to the 3' end of the nucleotide sequence, and (d) a 3' polyadenylation signal functionally coupled to the nucleotide sequence. Additional regulatory elements useful for enhancing or regulating gene expression or polypeptide processing may also be present. Promoters are genetic elements that are recognized by an RNA polymerase and mediate transcription of downstream regions. Preferred promoters are strong promoters that provide for increased levels of transcription. Examples of strong promoters are the immediate early human cytomegalovirus promoter (CMV), and CMV with intron A (Chapman et al, Nucl. Acids Res. 19:3979-3986, 1991). Additional examples of promoters include naturally occurring promoters such as the EF1 alpha promoter, the murine CMV promoter, Rous sarcoma virus promoter, and SV40 early/late promoters and the [beta]-actin promoter; and artificial promoters such as a synthetic muscle specific promoter and a chimeric muscle-specific/CMV promoter (Li et al., Nat. Biotechnol., 1999, 17:241-245; Hagstrom et al., Blood, 2000, 95:2536-2542).

The ribosome binding site is located at or near the initiation codon. Examples of preferred ribosome binding sites include CCACCAUGG, CCGCCAUGG, and ACCAUGG, where AUG is the initiation codon (Kozak, Cell, 1986, 44:283-292). The polyadenylation signal is responsible for cleaving the transcribed RNA and the addition of a poly (A) tail to the RNA. The polyadenylation signal in higher eukaryotes contains an AAUAAA sequence about 11-30 nucleotides from the polyadenylation addition site. The AAUAAA sequence is involved in signaling RNA cleavage (Lewin, Genes IV, Oxford University Press, NY, 1990). The poly (A) tail is important for the processing, export from the nucleus, translation and stability of the mRNA.

Polyadenylation signals that can be used as part of a gene expression cassette include the minimal rabbit [beta] -globin polyadenylation signal and the bovine growth hormone polyadenylation (BGH) (Xu et al., Gene, 2001, 272:149-156; Post et al., U.S. Patent 5,122,458).

Examples of additional regulatory elements useful for enhancing or regulating gene expression or polypeptide processing that may be present include an enhancer, a leader sequence and an operator. An enhancer region increases transcription. Examples of enhancer regions include the CMV enhancer and the SV40 enhancer (Hitt et al., Methods in Molecular Genetics, 1995, 7:13-30; Xu, et al., Gene, 2001, 272:149-156). An enhancer region can be associated with a promoter.

The expression of the protein according to the present invention may be regulated. Such regulation can be accomplished in many steps of the gene expression. Possible regulation steps are, for example but not limited to, initiation of transcription, promoter clearance, elongation of transcription, splicing, export from the nucleus, mRNA stability, initiation of translation, translational efficiency, elongation of translation and protein folding. Other regulation steps, which influence the concentration of a polypeptide inside a cell affect the half-life of the protein. Such a regulation step is, for example, the regulated degeneration of proteins.

The control of the regulatory steps mentioned above can be, for example, cell-type or tissue-type independent or cell-type or tissue-type specific. In a particularly preferred embodiment of the invention, the control of the regulatory steps is cell-type or tissue-type specific. Such a cell-type or tissue-type specific regulation is preferably accomplished through the regulation steps referring to the transcription of a nucleic acid. This transcriptional regulation can be accomplished through the use of cell-type or tissue-type specific promoter sequences. The result of this cell-type or tissue-type specific regulation can have different grades of specificity. This means, that the expression of a respective polypeptide is enhanced in the respective cell or tissue in comparison to other cell- or tissue-type or that the expression is limited to the respective cell- or tissue-type. Cell- or tissue-type specific promoter sequences are well known in the art and available for a broad range of cell- or tissue-types.

In another preferred embodiment, the expression is not cell-type or tissue-type specific but depends on physiological conditions. Such conditions are for example an inflammation or a wound. Such a physiological condition-specific expression can also be accomplished through regulation at all above mentioned regulation steps. The preferred way of regulation for a physiological condition-specific expression is the transcriptional regulation. For this purpose, a wound or inflammation specific promoter can be used. Respective promoters are, for example, natural occurring sequences, which can be, for example, derived from genes, which are specifically expressed during an immune reaction and/or the regeneration of wounded tissue. Another possibility is the use of artificial promoter sequences, which are, for example constructed through combination of two or more naturally occurring sequences.

In another preferred embodiment, the regulation is cell-type or tissue-type specific and physiological condition-specific. In a particularly preferred embodiment, the expression is a heart specific expression. In another particularly embodiment, the expression is heart specific and wound specific.

Another possibility for a regulation of expression of the protein according to the second aspect of the invention or the protein according to the second aspect of the invention is the conditional regulation of the gene expression. To accomplish conditional regulation, an operator sequence can be used. For example, the Tet operator sequence can be used to repress gene expression. The conditional regulation of gene expression by means of the Tet operator together with a Tet repressor is well known in the art and many respective systems have been established for a broad range of prokaryotic and eukaryotic organisms. A person of skill in the art knows how to choose a suitable system and adapt it to the special needs of the respective application.

In a particularly preferred embodiment the use of a nucleic acid according to the invention comprises the application to an individual specifically after myocardial infarction and the healing comprises the improvement of left ventricular systolic function and may be associated with an increase in capillary density in the infarct border zone. Additionally, it may reduce the mortality after a myocardial infarction. The methods which can be used to determine parameters like the improvement of left ventricular systolic function, increase in capillary density in the infarct border zone and the reduction of mortality after a myocardial infarction are well known in the art and exemplarily described in the examples section.

One or more nucleic acids encoding the polypeptide of the first aspect of the invention can be introduced into a host cell, a tissue or an individual using vectors suitable for therapeutic administration. Suitable vectors can preferably deliver nucleic acids into a target cell without causing an unacceptable side effect.

If the nucleic acid of the invention is a mRNA, in particular for use as a medicament, the delivery of mRNA therapeutics has been facilitated by significant progress in maximizing the translation and stability of mRNA, preventing its immune-stimulatory activity and the development of *in vivo* delivery technologies. The 5' cap and 3' poly(A) tail are the main contributors to efficient translation and prolonged half-life of mature eukaryotic mRNAs. Incorporation of cap analogs such as ARCA (anti-reverse cap analogs) and poly(A) tail of 120-150 bp into in vitro transcribed (IVT) mRNAs has markedly improved expression of the encoded proteins and mRNA stability. New types of cap analogs, such as 1,2-dithiodiphosphate-modified caps, with resistance against RNA decapping complex, can further improve the efficiency of RNA translation. Replacing rare codons within mRNA protein-coding sequences with synonymous frequently occurring codons, so-called codon optimization, also facilitates better efficacy of protein synthesis and limits mRNA destabilization by rare codons, thus preventing accelerated degradation of the transcript. Similarly, engineering 3' and 5' untranslated regions (UTRs), which contain sequences responsible for recruiting RNA-binding proteins (RBPs) and miRNAs, can enhance the level of protein product. Interestingly, UTRs can be deliberately modified to encode regulatory elements (e.g., K-turn motifs and miRNA binding sites), providing a means to control RNA expression in a cell-specific manner. Some RNA base modifications such as N1-methyl-pseudouridine have not only been instrumental in masking mRNA immune-stimulatory activity but have also been shown to increase mRNA translation by enhancing translation initiation. In addition to their observed effects on protein translation, base modifications and codon optimization affect the secondary structure of mRNA, which in turn influences its translation. Respective modifications of the nucleic acid molecules of the invention are also contemplated by the invention.

The RNA or a plurality of RNAs preferably encode the polypeptide of the invention. Specific methods for delivering and expressing nucleic acids and specifically RNAs are disclosed e.g. in EP2590676 and EP3115064. The RNA may be present in a particle and is preferably self-replicating. After *in vivo* administration of the particles, RNA is released from the particles and is translated inside a cell to provide the DNA recombining enzyme or any of its monomeric subunits.

A self-replicating RNA molecule (replicon) can, when delivered to a vertebrate cell even without any proteins, lead to the production of multiple daughter RNAs by transcription from itself (via an antisense copy which it generates from itself). These daughter RNAs, as well as collinear subgenomic transcripts, may be translated by themselves to provide in situ expression of an encoded polypeptide, or may be transcribed to provide further transcripts with the same sense as the delivered RNA which are translated to provide in situ expression of the polypeptide. The overall results of this sequence of transcriptions is a huge amplification in the number of the introduced replicon RNAs and so the encoded polypeptide becomes a major polypeptide product of the cells.

A preferred self-replicating RNA molecule in accordance with the invention encodes (i) a RNA-dependent RNA polymerase which can transcribe RNA from the self-replicating RNA molecule and (ii) a polypeptide of the present invention. The polymerase can be an alphavirus replicase e.g. comprising one or more of alphavirus proteins nsP 1, nsP2, nsP3 and nsP4. It is preferred that the self-replicating RNA molecules of the invention do not encode alphavirus structural proteins. Thus a preferred self-replicating RNA can lead to the production of genomic RNA copies of itself in a cell, but not to the production of RNA-containing virions. A self-replicating RNA molecule useful in the context of the present invention may have two open reading frames. The first (5') open reading frame encodes a replicase, and the second (3') open reading frame encodes a polypeptide of the present invention. In some embodiments the RNA may have additional (e.g. downstream) open reading frames e.g. for further encoding accessory polypeptides.

Such RNA is particularly suitable for the general use in gene therapy, and specifically for use in the treatment of the disorders or diseases described herein.

The present invention further provides a vector comprising a nucleic acid encoding the polypeptide of the present invention. The vector is specifically for use in treating or preventing the conditions discussed herein.

In a preferred embodiment of the invention, the vector is a viral vector. Suitable viral vectors include but are not limited to adenoviral vectors, adeno-associated viral (AAV) vectors, alphaviral vectors, herpes viral vectors, measles viral vectors, pox viral vectors, vesicular stomatitis viral vectors, retroviral vector and lentiviral vectors. In a particularly preferred embodiment of the invention, the vector is an adenoviral or an adeno-associated viral (AAV) vector.

In a particularly preferred embodiment, the use of a vector according to the invention comprises the application to an individual after myocardial infarction. According to a preferred embodiment, the vector is administered via a route selected from the group consisting of intravenous, intraarterial, intramuscular, subcutaneous, transdermal, intrapulmonary, intraperitoneal, intracoronary, intracardiac administration, or administration via mucous membranes. According to a specifically preferred embodiment, the vector is administered intracoronarily or intracardiacally.

The present invention further provides a host cell comprising the nucleic acid or nucleic acids of the present invention or the vector of the present invention. The term "host cell" as used herein also encompasses a culture of host cells. The host cell or culture of host cells is specifically for use in treating or preventing the conditions discussed herein. The host cell or culture of host cells preferably expresses the nucleic acid or nucleic acids encoding the polypeptide of the invention. A host cell in accordance with the present invention may either be a prokaryotic (e.g. a bacterial cell) or a eukaryotic cell (e.g. a fungal, plant or animal cell). Methods for genome integration of recombinant DNA into respective host cells, such as homologous recombination or transposase-mediated integration, are well known in the art. The host cells can be cultured in conventional nutrient media modified as appropriate for activating promoters, selecting transformants or amplifying the genes encoding the polypeptide of the present invention. The culture conditions, such as temperature, pH and the like, are those commonly used with the host cell selected for expression, and are well known to the skilled person.

The present invention further provides a pharmaceutical composition comprising the polypeptide, the nucleic acid or nucleic acids, the vector, or the host cell or culture of host cells of the present invention, and optionally a suitable pharmaceutical excipient. The pharmaceutical composition is specifically for use in treating or preventing the conditions discussed herein. Therefore, according to a preferred embodiment of the invention, the pharmaceutical compositions is customized for the treatment of a disease or disorder disclosed herein.

The pharmaceutical composition can be formulated in various ways well known to one of skill in the art. For example, the pharmaceutical composition of the present invention may be in liquid form such as in the form of solutions, emulsions, or suspensions. Preferably, the pharmaceutical composition of the present invention is formulated for parenteral administration, preferably for intravenous, intraarterial, intramuscular, subcutaneous, transdermal, intrapulmonary, intraperitoneal intracoronary, intracardiac administration, or administration via mucous membranes, preferably for intracoronary or intracardiac administration. A preparation for oral or anal administration is also possible. Preferably, the pharmaceutical composition of the present invention is in the form of a sterile aqueous solution which may contain other substances, for example, enough salts or glucose to make the solution isotonic with blood. The aqueous solutions can be suitably buffered (preferably to a pH of from 3 to 9, more preferably to a pH of from 5 to 7). The pharmaceutical composition is preferably in unit dosage form. In such form, the pharmaceutical composition is subdivided into unit doses containing appropriate quantities of the active component. The unit dosage form can be a packaged preparation, the package containing discrete quantities of pharmaceutical composition such as vials or ampoules.

The pharmaceutical composition is preferably administered through the intravenous, intraarterial, intraperitoneal, intracoronary or intracardiac route, wherein other routes of administration known in the art are also comprised.

In case the pharmaceutical composition is used as a treatment for an individual, the use of the pharmaceutical composition can replace the standard treatment for the respective disease or condition or can be administered additionally to the standard treatment. In the case of an additional use of the pharmaceutical composition, the pharmaceutical composition can be administered before, simultaneously or after a standard therapy. In a preferred embodiment, the standard therapy is a reperfusion therapy and the pharmaceutical composition can be administered before, simultaneously or after the reperfusion therapy.

It is further preferred that the pharmaceutical composition is administered once or more than once. This comprises 2, 3, 4, 5, 6, 7, 8, 9, 10, 11, 12, 13, 14, 15, 16, 17, 18, 19, 20, 25, 30, 35, 40, 45 or 50 times. The time span for the administration of the pharmaceutical is not limited. Preferably, the administration does not exceed 1, 2, 3, 4, 5, 6, 7 or 8 weeks.

A single dose of the pharmaceutical composition, can be independently from the overall number of administered doses or the respective time span of administration. The pharmaceutical composition can be administered as one or more bolus injection(s) and/or infusion(s).

The present invention also relates to respective methods for treatment or prevention of adverse conditions of the heart, specifically those disclosed herein. Specifically, the present invention provides methods of treating or preventing heart failure, myocardial infarction or hypertrophy, reducing infarct size or scar size after myocardial infarction, inducing endothelial cell sprouting after myocardial infarction, protecting cells from ischemia- or reperfusion-induced injury, promoting angiogenesis after myocardial infarction, promoting coronary endothelial cell migration and proliferation, inhibiting cardiomyocyte hypertrophy, or inhibiting inflammation. The method comprises administering to a patient in need thereof a therapeutically active amount of the polypeptide of the present invention or of the fragment or the variant thereof, as defined herein.

The present invention further provides methods of treating or preventing heart failure, myocardial infarction or hypertrophy, reducing infarct size or scar size after myocardial infarction, inducing endothelial cell sprouting after myocardial infarction, protecting cells from ischemia- or reperfusion-induced injury, promoting angiogenesis after myocardial infarction, promoting coronary endothelial cell migration and proliferation, inhibiting cardiomyocyte hypertrophy, or inhibiting inflammation. The method comprises administering to a patient in need thereof a therapeutically active amount of the nucleic acid or nucleic acids of the present invention, as defined herein

The present invention further provides methods of treating or preventing heart failure, myocardial infarction or hypertrophy, reducing infarct size or scar size after myocardial infarction, inducing endothelial cell sprouting after myocardial infarction, protecting cells from ischemia- or reperfusion-induced injury, promoting angiogenesis after myocardial infarction, promoting coronary endothelial cell migration and proliferation, inhibiting cardiomyocyte hypertrophy, or inhibiting inflammation. The method comprises administering to a patient in need thereof a therapeutically active amount of the vector of the present invention, as defined herein.

The present invention further provides methods of treating or preventing heart failure, myocardial infarction or hypertrophy, reducing infarct size or scar size after myocardial infarction, inducing endothelial cell sprouting after myocardial infarction, protecting cells from ischemia- or reperfusion-induced injury, promoting angiogenesis after myocardial infarction, promoting coronary endothelial cell migration and proliferation, inhibiting cardiomyocyte hypertrophy, or inhibiting inflammation. The method comprises administering to a patient in need thereof a therapeutically active amount of the host cell or culture of host cells of the present invention, as defined herein.

The present invention further provides methods of treating or preventing heart failure, myocardial infarction or hypertrophy, reducing infarct size or scar size after myocardial infarction, inducing endothelial cell sprouting after myocardial infarction, protecting cells from ischemia- or reperfusion-induced injury, promoting angiogenesis after myocardial infarction, promoting coronary endothelial cell migration and proliferation, inhibiting cardiomyocyte hypertrophy, or inhibiting inflammation. The method comprises administering to a patient in need thereof a therapeutically active amount of the pharmaceutical composition of the present invention, as defined herein.

### Examples

The following examples are provided for the sole purpose of illustrating various embodiments of the present invention and are not meant to limit the present invention in any way.

### Example 1: Screening study, in vitro screen

In a multi-centre, placebo-controlled clinical trial the effects of an intracoronary infusion of autologous bone marrow cells in patients with AMI were tested by one of the inventors (BOOST-2, Controlled Trials Identification No. ISRCTN17457407). Within the trial, bone marrow aspirates were obtained from AMI patients for research purposes. CXCR4⁺ bone marrow cells were isolated by magnetic cell separation (MiniMACS ^{™}, Miltenyi Biotec). After two subsequent purification steps, a CXCR4⁺ enriched cell population was obtained (>95% purity as confirmed by flow cytometry). Next, RNA was isolated from these cells and the RNA was used in a microarray analysis (Affymetrix GeneChip HG_U133 Plus 2.0). In a subsequent bioinformatic analysis, the 4,000 expressed sequence tags (ESTs) that were most strongly expressed by CXCR4⁺ bone marrow cells in the microarray were examined. A series of bioinformatic tools was used to identify putative secreted factors among those ESTs that were either predicted to be classically secreted (based on an N-terminal signal peptide) or to be non-classically secreted, and to have no mitochondrial or nuclear signal peptides, no endoplasmic reticulum retention sequence, and no transmembrane domains. In total, 283 putative secreted factors were identified; 117 of those were found in NCBI Blast to have a mouse homologue. The cDNAs of the human homologues were cloned into expression plasmids which were then individually transfected into human embryonic kidney (HEK) cells. The transfected HEK cells were cultured in serum-free medium to obtain conditioned culture supernatants after 30 hours. The conditioned HEK cell supernatants were individually tested for pro-angiogenic effects in miniaturized angiogenesis assays and cytoprotective effects in cardiomyocyte cell death assays. This screen resulted in the identification of candidate factors that protected serum-starved neonatal rat ventricular cardiomyocytes from cell death and/or promoted angiogenic effects in human coronary artery endothelial cells: MYDGF (UniProt: Q969H8), ER membrane protein complex subunit 10 (EMC10; UniProt: Q5UCC4), BRICK1 (UniProt: Q8WUW1), FAM163A (UniProt: Q96GL9), FAM168A (UniProt: Q92567), Shieldin complex subunit 1 (SHLD1; C20orf196; UniProt: Q8IYI0), and Podocan-like protein 1 (PODNL1; UniProt: Q6PEZ8).

### Example 2: Adenoviral in vivo screen

To examine the therapeutic potential of the candidate factors identified in Example 1 *in vivo,* adenoviruses (Ad) encoding each of these individual factors were generated. As positive controls, adenoviruses encoding the cardioprotective factors MYDGF or EMC10 (Korf-Klingebiel M et al., Nat Med. 2015; 21:140-149; Reboll MR et al., Circulation. 2017; 136:1809-1823), were also constructed. An adenovirus encoding beta-galactosidase was used as negative control (Ad.Con). Adenoviruses were generated using the AdEasy Adenoviral Vector System (Agilent, cat. no. 240009). Mouse BRICK1 cDNA (NM_133937.1; SEQ ID NO: 4) was used. 8 to 10-week-old male C57BL/6 mice were subjected to transient left anterior descending coronary artery ligation (ischemia) for one hour. This model simulates the situation in patients with acute myocardial infarction (MI) undergoing reperfusion therapy. One week before surgery, mice received a first adenovirus injection (into the tail vein). Immediately after surgery, mice received a second adenovirus injection into the left ventricular (LV) cavity. After 28 days, LV end-diastolic area (LVEDA) and LV end-systolic area (LVESA) were recorded by high-resolution echocardiography (n= 5 to 15 mice per group). As a measure of systolic function, fractional area change was calculated from these data as [(LVEDA -LVESA) / LVEDA] × 100%.

The adenovirus encoding BRICK1 was found to improve heart function after MI. The magnitude of this beneficial effect was comparable to the beneficial effects of the adenoviruses encoding MYDGF or EMC10. In contrast, adenoviruses encoding the other candidates from the *in vitro* screen (Example 1) did not exert any therapeutic effects post MI (Fig. 1). *P<0.05, **P<0.01 vs. Ad.Con.

### Example 3: Adenoviral in vivo screen identifies BRICK1 as a protein that reduces scar size after acute myocardial infarction

As a second endpoint indicating therapeutic potential, left ventricular (LV) scar size was determined by Masson's trichrome staining 28 days after reperfusion (same animals and experimental setup as in Fig. 1). Summary data from n = 4 to 13 mice per group are presented in Fig. 2. Scar size is shown as percentage area of left ventricle. *P<0.05, **P<0.01 vs. Ad.Con.

The adenovirus encoding mouse BRICK1 was found to reduce scar size as effectively as the adenovirus encoding MYDGF. In contrast, adenoviruses encoding the other four candidates from the *in vitro* screen (Example 1) did not exert any therapeutic effects. Based on these data, BRICK1 was chosen for an in-depth analysis of its therapeutic potential after acute MI and in chronic HF.

### Example 4: BRICK1 protects neonatal rat ventricular cardiomyocytes (NRCM) from simulated ischemia/reperfusion (I/R)-induced cell death

Ventricular cardiomyocytes were isolated from 1 to 3-day-old Sprague-Dawley rats, cultured in the absence (control, Con) or presence of recombinant mouse BRICK1 (1, 3, 10, 30 and 100 ng/mL doses) or myeloid-derived growth factor (MYDGF, 100 ng/mL dose), and were exposed to simulated ischemia (glucose-free medium containing 2-deoxyglucose in an atmosphere with 5% CO₂ and 95% N₂). After 180 minutes, reperfusion was simulated by switching the cells back to glucose-containing medium in 5% CO₂ and 95% room air. After 60 minutes of reperfusion, apoptotic cell death was assessed by in situ TdT-mediated dUTP nick end-labeling (TUNEL). n=3 experiments. Results are shown in Fig. 3. *P<0.05 vs. I/R Con.

### Example 5: BRICK1 protein therapy reduces infarct size and protects cardiomyocytes and endothelial cells after myocardial infarction

8 to 10-week-old male FVB/N mice were subjected to transient left anterior descending coronary artery ligation (ischemia) for one hour. This model simulates the situation in patients with acute MI undergoing reperfusion therapy. At the time of reperfusion, mice received an intra-left ventricular (LV) cavity bolus injection of recombinant mouse BRICK1 (10 µg) or PBS as control. Subsequently, mice received a 24 h subcutaneous infusion of 10 µg recombinant mouse BRICK1 or PBS (control) using osmotic minipumps placed in a subcutaneous interscapular pocket. After 24 h, area-at-risk (AAR) and infarct size were determined by Evans blue and 2,3,5-triphenyltetrazolium chloride staining of basal, midventricular, and apical LV tissue slices. Representative tissue sections are shown in Fig. 4A, with infarct areas shown in the periphery as bright color. A summary of the infarct size data is shown in Fig. 4B (n=8 control, n=6 BRICK1-treated mice). In addition, the number of TUNEL⁺ (apoptotic) cardiac myocyte (CM) nuclei in the infarct border zone 24 h after reperfusion (Fig. 4C) (n=3-4 mice per group) and the number of TUNEL⁺ (apoptotic) IB4⁺ endothelial cell nuclei in the infarct border zone 24 h after reperfusion (n=3-4 mice per group) (Fig. 4D) have been evaluated. *P<0.05, **P<0.01.

### Example 6: BRICK1 protects human coronary artery endothelial cells from simulated ischemia/reperfusion (I/R)-induced cell death

Human coronary artery endothelial cells (HCAECs) were cultured in the absence (control) or presence of recombinant human BRICK1 (100 ng/mL) or vascular endothelial growth factor (VEGFA, 50 ng/ml) and were exposed to simulated ischemia (glucose-free medium containing 2-deoxyglucose in an atmosphere with 5% CO₂ and 95% N₂). After 180 minutes, reperfusion was simulated by switching the cells back to glucose-containing medium in 5% CO₂ and 95% room air. After 60 minutes of reperfusion, apoptotic cell death was assessed by in situ TdT-mediated dUTP nick end-labeling (TUNEL, n=3 experiments). Results are shown in Fig. 5A (exemplary images; DAPI, blue; TUNEL, red) and Fig. 5B (summary data). ***P<0.001 vs. I/R Con.

### Example 7: BRICK1 promotes human coronary artery endothelial cell migration and proliferation

Human coronary artery endothelial cells (HCAECs) were cultured in the absence (control) or presence of recombinant human BRICK1 (1, 3, 10, 30, 100, 300 and 1000 ng/mL) or vascular endothelial growth factor (VEGFA, 50 ng/mL). Control cells in cell medium (MCDB131 [Invitrogen] supplemented with 1% fetal calf serum) only. For the cell migration assay, confluent HCAEC monolayers were scratched with a pipet tip. Microscopic images were taken at baseline (0 h) and after 16 h. Recovery rate (%) was calculated as ([cell free area at 0 h - cell free area at 16 h] / cell free area at 0 h) × 100; n=4 experiments. Results are shown in Fig. 6A. As a measure for cell proliferation, BrdU incorporation was determined after stimulation with the indicated doses and after 48h; n=4 experiments. Results are shown in Fig. 6B. *P<0.05, **P<0.01, ***P<0.001 vs. unstimulated Control.

### Example 8: BRICK1 induces endothelial cell sprouting from myocardial infarct explants

Myocardial infarction was induced by transient left anterior descending coronary artery ligation (ischemia) for one hour in Tg(TIE2GFP)287Sato/J mice expressing green fluorescent protein (GFP) in endothelial cells under the control of the Tie2 promoter. After 24 h, approximately 1 mm³ samples from the infarcted and non-infarcted regions of the left ventricle were obtained and cultured for seven days in 48-well plates (1 sample per well) on growth factor-reduced Matrigel in the absence (control) or presence of recombinant mouse BRICK1 (100 ng/mL) or vascular endothelial growth factor (VEGFA, 50 ng/mL). Microscopic images are shown in Fig. 7A (TL, transmitted light; GFP fluorescence), a graphic summary of the observed results is shown in Fig. 7B; n=5 explants per group. ***P<0.001 vs. unstimulated Control.

### Example 9: BRICK1 induces phosphoproteome changes in human coronary artery endothelial cells (HCAECs) associated with cell viability and activation

Human coronary artery endothelial cells (HCAEC) were cultured in the absence (control) or presence of recombinant human BRICK1 (100 ng/mL) for 15 min and subjected to phosphoproteome analysis (n=4 experiments). Responsive phosphosites (P<0.05) were used for functional enrichment analysis using Ingenuity Pathway Analysis software (Qiagen) and the Qiagen knowledgebase and annotation sets. Enriched functions were filtered for data set size-adjusted enrichment (P<0.05). The z-score indicates activation (z-score>0) or inhibition (z-score<0) based on the comparison of observed phosphosite regulation with the expected function activity pattern based on previous experimental data from the literature; z-scores>2 or <-2 are considered strongly activated or inhibited, respectively. The sizes of the black circles indicate the number of phosphosites associated with the respective biological term. The results show that BRICK1 induces phosphoproteome changes most strongly associated with cell survival and viability, and at the same time reduced necrosis, cell death, and apoptosis (Fig. 8).

### Example 10: BRICK1 protein therapy promotes angiogenesis and reduces scar size after myocardial infarction

8 to 10-week-old male FVB/N mice were subjected to transient left anterior descending coronary artery ligation (ischemia) for one hour. This model simulates the situation in patients with acute MI undergoing reperfusion therapy. At the time of reperfusion, mice received an intra-left ventricular (LV) cavity bolus injection of recombinant mouse BRICK1 (10 µg); PBS was injected in control mice. Subsequently, mice received a subcutaneous infusion of recombinant mouse BRICK1 (10 µg/day for 7 days) or PBS (control) using osmotic minipumps placed in a subcutaneous interscapular pocket. Fig. 9A shows the number of isolectin B4⁺ cells (green) per cardiomyocyte (CM) in the infarct border zone quantified at 2, 6, and 28 days (D2, D6, D28) after reperfusion in fluorescent microscopy images (red, Masson's trichrome). Fig. 9B shows a graphic summary of the data obtained from the microscopic images. *P<0.05, **P<0.01 vs. untreated, sham-operated mice. ^{#}P<0.05, ^{###}P<0.001 BRICK-treated vs. PBS-treated infarcted control (Con) mice.

In addition, LV scar size was determined by Masson's trichrome staining 28 days after reperfusion Representative images are shown in Fig. 9C. A graphic summary of the data is shown in Fig. 9D. *P<0.05 BRICK-treated infarcted mice vs. PBS-treated infarcted control (Con) mice.

### Example 11: BRICK1 protein therapy attenuates adverse left ventricular (LV) remodeling and improves LV function after myocardial infarction

8 to 10-week-old male FVB/N mice were subjected to transient left anterior descending coronary artery ligation (ischemia) for one hour. This model simulates the situation in patients with acute MI undergoing reperfusion therapy. At the time of reperfusion, mice received an intra-LV cavity bolus injection of recombinant mouse BRICK1 (10 µg); PBS was injected in control mice. Subsequently, mice received a subcutaneous infusion of recombinant mouse BRICK1 (10 µg/day for 7 days) or PBS (control) using osmotic minipumps placed in a subcutaneous interscapular pocket (n=5-11 mice per group). After 6 and 28 days, LV end-diastolic area (LVEDA) and LV end-systolic area (LVESA) were recorded by high-resolution echocardiography as measures of LV remodeling (Fig. 10A). As a measure of systolic function, fractional area change (FAC) was calculated from these data as [(LVEDA - LVESA) / LVEDA] × 100% (Fig. 10B). ***P<0.001 vs. all infarcted groups. ^{###}P<0.001 BRICK-treated infarcted mice vs. PBS-treated infarcted control (Con) mice.

### Example 12: BRICK1 protein therapy dose-dependently attenuates cardiomyocyte injury and left ventricular (LV) dysfunction after myocardial infarction

8 to 10-week-old male FVB/N mice were subjected to transient left anterior descending coronary artery ligation (ischemia) for one hour. At the time of reperfusion, mice received an intra-LV cavity bolus injection of PBS (control mice) or recombinant mouse BRICK1 (1, 3, 10, 30, or 100 µg). Subsequently, mice received a subcutaneous infusion of PBS (control) or recombinant mouse BRICK1 (1, 3, 10, 30, or 100 µg/day for 24 h (Fig. 11A) or 7 days (Fig. 11B)) using osmotic minipumps placed in a subcutaneous interscapular pocket (n=4-9 mice per group). EDTA-treated blood samples were collected from the facial vein 24 h after reperfusion (Fig. 11A). Samples were immediately centrifuged and high-sensitivity cardiac troponin T levels were measured with an Elecsys assay (Roche Diagnostics). *P<0.05 vs. PBS-treated infarcted control mice. After 28 days, fractional area change (FAC) was determined by 2D echocardiography (Fig. 11B). *P<0.05, **P<0.01 vs. PBS-treated infarcted control mice. For comparison, FAC in sham-operated (non-infarcted) mice is indicated with a dashed line.

### Example 13: Mouse and human BRICK1 protein therapy attenuates left ventricular (LV) dysfunction after myocardial infarction

An alignment of the amino acid sequences of human BRICK1 and mouse BRICK1 is shown in Fig. 12A.

8 to 10-week-old male FVB/N mice were subjected to transient left anterior descending coronary artery ligation (ischemia) for one hour. At the time of reperfusion, mice received an intra-LV cavity bolus injection of PBS (control mice) or recombinant mouse or human BRICK1 (10 µg). Subsequently, mice received a subcutaneous infusion of PBS (control) or recombinant mouse or human BRICK1 (10 µg/day for 7 days) using osmotic minipumps placed in a subcutaneous interscapular pocket (n=7-8 mice per group). After 6 and 28 days, fractional area change (FAC) was determined by 2D echocardiography (Fig. 12B). ^{#}P<0.05, ^{###}P<0.001 BRICK-treated infarcted mice vs. PBS-treated infarcted control (Con) mice. FAC in sham-operated mice is shown as well.

### Example 14: BRICK1 inhibits neonatal rat ventricular cardiomyocyte hypertrophy

Neonatal rat ventricular cardiomyocytes (NRCMs) were isolated from 1 to 3-day-old Sprague-Dawley rats and cultured in the absence (control) or presence of recombinant mouse BRICK1 (100 ng/mL) or the pro-hypertrophic agonist, endothelin 1 (ET-1, 100 nmol/L) for 24 h. Cell size was assessed by planimetry after 24 h. n=5 experiments (Fig. 13A). ^{∗∗∗}P<0.001 vs. unstimulated control cells. ^{###}P<0.001. Expression levels of *Nppa* mRNA (encoding, natriuretic peptide type A) and *Myh7* mRNA (encoding, beta myosin heavy chain) - marker genes known to be activated in maladaptive hypertrophy - were determined after 24 h by RT-qPCR (Fig. 13B). n=3 experiments. ***P<0.001 vs. unstimulated control cells. ^{##}P<0.01. BRICK1 effectively reduced cell surface area in a hypertrophy stimulation environment and effectively reduced expression of maladaptive hypertrophy marker genes.

### Example 15: BRICK1 protein therapy attenuates left ventricular (LV) dysfunction after transverse aortic constriction (TAC)-induced LV hypertrophy and heart failure

6 to 8-week-old male C57BL/6 mice were subjected to transverse aortic constriction (TAC) surgery to induce pressure overload, LV hypertrophy, and heart failure. Immediately after TAC, mice received an intraperitoneal injection of PBS (control mice) or recombinant mouse BRICK1 (10 µg). Subsequently, mice received a subcutaneous infusion of PBS (control) or recombinant mouse BRICK1 (10 µg/day for 7 days) using osmotic minipumps placed in a subcutaneous interscapular pocket. At baseline and after 7 and 42 days, fractional area change (FAC) was determined by 2D echocardiography (Fig. 14). ***P<0.001 vs. all TAC groups; ^{#}P<0.05.

### Example 16: BRICK1 inhibits inflammation after myocardial infarction

8 to 10-week-old male FVB/N mice were subjected to transient left anterior descending coronary artery ligation (ischemia) for one hour. This model simulates the situation in patients with acute MI undergoing reperfusion therapy. At the time of reperfusion, mice received an intra-LV cavity bolus injection of recombinant mouse BRICK1 (10 µg); PBS was injected in infarcted and reperfused control mice. Subsequently, mice received a subcutaneous infusion of recombinant mouse BRICK1 (10 µg/day for 7 days) or PBS (control) using osmotic minipumps placed in a subcutaneous interscapular pocket (n=4 mice per group and time point). 1, 3, and 7 days after reperfusion, accumulation of neutrophils, Ly6C^{high} monocytes, Ly6C^{low} monocytes or macrophages, T cells, and B cells in the infarct region was assessed by flow cytometry (following previously described protocols for cell isolation and flow cytometry: Korf-Klingebiel M et al., Nat Med. 2015; 21: 140-149) (Fig. 15). *P<0.05 vs. same-day control group.

### Example 17: BRICK1 inhibits human myeloid cell migration along chemotactic gradients

Transendothelial THP-1 cell migration was assessed in a Transwell assay (Lifesciences, cat. no. 3421). THP-1 cells are a human monocytic (myeloid) cell line. In the upper chamber, a confluent HCAEC layer was grown on a filter membrane (5 µm pore size). 50,000 THP-1 cells were then added to the upper chamber and exposed for 2 hours to BRICK1 (100 ng/mL) or diluent-only (control). The upper chamber (containing BRICK1 or diluent-only and THP-1 cells on top of the HCAEC monolayer) was then transferred to the Transwell plate and chemoattractants were added to the bottom chamber (C-X-C motif chemokine 12 [CXCL12], stem cell factor [SCF], or C-C motif chemokine 2 [CCL2]; 100 ng/mL each). After 16 hours, THP-1 cells transmigrated through the HCAEC monolayer along the chemotactic gradient into the bottom chamber were counted. Migration is expressed as fold-increase vs. no chemokine gradient (Fig. 16). *P<0.05.

## Claims

1. A polypeptide comprising the amino acid sequence according to SEQ ID NO: 1 or a variant thereof, or a fragment of SEQ ID NO: 1 or of the variant thereof, for use in treating or preventing heart failure, myocardial infarction or hypertrophy, wherein the variant has at least 80% sequence identity to SEQ ID NO: 1, and wherein the fragment comprises at least amino acids 14 to 75 of SEQ ID NO: 1 or of the variant thereof.

2. The polypeptide for use of claim 1, wherein the fragment or the variant exhibits the biological function of BRICK1.

3. The polypeptide for use of claim 1 or 2, wherein the heart failure is chronic heart failure.

4. A polypeptide comprising the amino acid sequence according to SEQ ID NO: 1 or a variant thereof, or a fragment of SEQ ID NO: 1 or of the variant thereof, for use in
reducing infarct size or scar size after myocardial infarction,
inducing endothelial cell expansion,
protecting cells from ischemia- or reperfusion-induced injury,
promoting angiogenesis after myocardial infarction,
promoting coronary endothelial cell migration and proliferation,
inhibiting cardiomyocyte hypertrophy, or
inhibiting inflammation,
wherein the variant has at least 80% sequence identity to SEQ ID NO: 1, and wherein the fragment comprises at least amino acids 14 to 75 of SEQ ID NO: 1 or of the variant thereof.

5. The polypeptide for use of claim 4, wherein the fragment or the variant exhibits the biological function of BRICK1.

6. The polypeptide for use according to any one of claims 1 to 5, wherein:
(i) the polypeptide consists of SEQ ID NO: 1;
(ii) the variant of SEQ ID NO: 1 has at least 90% sequence identity to SEQ ID NO: 1 and exhibits the biological function of BRICK1;
(iii) the variant has at least 50% of the biological activity of BRICK 1; or
(iv) a fragment of (i) to (iii) comprising at least amino acids 14 to 75.

7. The polypeptide for use according to any one of claims 4 to 6, wherein the cells to be protected from ischemia- or reperfusion-induced injury are cardiomyocytes and/or coronary endothelial cells.

8. The polypeptide for use according to any one of claims 1 to 7, wherein the polypeptide is a recombinant polypeptide.

9. A nucleic acid encoding the polypeptide for use of claims 1 to 8, for use in
treating or preventing heart failure, myocardial infarction or hypertrophy, or for use in reducing infarct size or scar size after myocardial infarction,
inducing endothelial cell expansion after myocardial infarction,
protecting cells from ischemia- or reperfusion-induced injury,
promoting angiogenesis after myocardial infarction,
promoting coronary endothelial cell migration and proliferation,
inhibiting cardiomyocyte hypertrophy, or
inhibiting inflammation.

10. A vector comprising a nucleic acid encoding the polypeptide for use of claims 1 to 8, for use in treating or preventing heart failure, myocardial infarction or hypertrophy, or for use in
reducing infarct size or scar size after myocardial infarction,
inducing endothelial cell sprouting after myocardial infarction,
protecting cells from ischemia- or reperfusion-induced injury,
promoting angiogenesis after myocardial infarction,
promoting coronary endothelial cell migration and proliferation,
inhibiting cardiomyocyte hypertrophy, or
inhibiting inflammation.

11. The vector for use according to claim 10, wherein the vector is an adenoviral vector.

12. A host cell comprising the nucleic acid for use according to claim 9, or the vector for use according to any one of claims 10 or 11, for use in
treating or preventing heart failure, myocardial infarction or hypertrophy,
reducing infarct size or scar size after myocardial infarction,
inducing endothelial cell sprouting after myocardial infarction,
protecting cells from ischemia- or reperfusion-induced injury,
promoting angiogenesis after myocardial infarction,
promoting coronary endothelial cell migration and proliferation,
inhibiting cardiomyocyte hypertrophy, or
inhibiting inflammation.

13. A pharmaceutical composition comprising the polypeptide for use according to any one of claims 1 to 8, the nucleic acid for use according to claim 9, the vector for use according to any one of claims 10 or 11, or the host cell for use according to claim 12, and optionally a suitable pharmaceutical excipient, for use in
treating or preventing heart failure, myocardial infarction or hypertrophy,
reducing infarct size or scar size after myocardial infarction,
inducing endothelial cell sprouting after myocardial infarction,
protecting cells from ischemia or reperfusion-induced injury,
promoting angiogenesis after myocardial infarction,
promoting coronary endothelial cell migration and proliferation,
inhibiting cardiomyocyte hypertrophy, or
inhibiting inflammation.

14. The pharmaceutical composition for use of claim 13, wherein said pharmaceutical composition is administered through the oral, intravenous, subcutaneous, intramucosal, intraarterial, intramuscular or intracoronary route.

15. The pharmaceutical composition for use of claim 14, wherein the administration is through one or more bolus injection(s) and/or infusion(s).
